# EUROPEAN PATENT SPECIFICATION

(11) **EP 1 756 110 B1**
(45) Date of publication and mention of the grant of the patent: **31.03.2010**
(21) Application number: 05739554.3
(22) Date of filing: 25.04.2005
(51) Int. Cl.: C07D 487/04, A61K 31/4985, A61P 29/00

(54) **7H-PYRROLO[2,3-d]PYRIMIDINE DERIVATIVES, AS WELL AS THEIR THERAPEUTICALLY ACCEPTABLE SALTS, PHARMACEUTICAL PREPARATIONS CONTAINING THEM AND PROCESS FOR PRODUCTION THE ACTIVE AGENT**
7H-PYRROLO[2,3-d]PYRIMIDINDERIVATE SOWIE DEREN THERAPEUTISCH ANNEHMBARE SALZE, PHARMAZEUTISCHE ZUBEREITUNGEN, DIE DIESE ENTHALTEN, UND VERFAHREN ZUR HERSTELLUNG DES WIRKSTOFFS
DERIVES DE 7H-PYRROLO[2,3-d]PYRIMIDINE ET LEURS SELS ACCEPTABLES D'UN POINT DE VUE THERAPEUTIQUE, PREPARATIONS PHARMACEUTIQUES LES CONTENANT ET PROCEDE DE PRODUCTION DE L'AGENT ACTIF

(30) Priority: 29.04.2004 HU 0400891
(43) Date of publication of application: 28.02.2007
(73) Proprietor: Szolcsányi, János, 7624 Pécs (HU); Orfi, László, 1161 Budapest (HU); Kéri, György, 1021 Budapest (HU); Wáczek, Frigyes, 1195 Budapest (HU); Pintér, Erika, 7624 Pécs (HU); Helyes, Zsuzsanna, 7629 Pécs (HU); Szüts, Tamás, 1122 Budapest (HU); Németh, József, 7623 Pécs (HU)
(72) Inventor: Szolcsányi, János, 7624 Pécs (HU); Orfi, László, 1161 Budapest (HU); Kéri, György, 1021 Budapest (HU); Wáczek, Frigyes, 1195 Budapest (HU); Pintér, Erika, 7624 Pécs (HU); Helyes, Zsuzsanna, 7629 Pécs (HU); Szüts, Tamás, 1122 Budapest (HU); Németh, József, 7623 Pécs (HU)
(74) Representative: Ravadits, Imre
(86) International application number: PCT/HU2005/000040
(87) International publication number: WO 2005/105804

(56) References cited:
- EP-A- 1 081 149
- WO-A-01/42246
- US-A- 4 229 453
- W.M. BASYOUNI, H.M. HOSNI, K.A.M. EL-BAYOUKI: "Pyrrolo[2,3-d]pyrimidines. Part 3: Synthesis of some novel 4-substituted pyrrolo[2,3-d]pyrimidines and their related derivatives" JOURNAL OF CHEMICAL RESEARCH, MINIPRINT, vol. 12, 1997, pages 2771-2789, XP009054934 cited in the application
- TRAXLER P M ET AL: "4-(PHENYLAMINO)PYRROLOPYRIMIDINES: POTENT AND SELECTIVE, ATP SITE DIRECTED INHIBITORS OF THE EGF-RECEPTOR PROTEIN TYROSINE KINASE" JOURNAL OF MEDICINAL CHEMISTRY, AMERICAN CHEMICAL SOCIETY. WASHINGTON, US, vol. 39, no. 12, December 1996 (1996-12), pages 2285-2292, XP002053503 ISSN: 0022-2623 cited in the application

## Description

The invention relates to new 7H-pyrrolo[2,3-d]pyrimidine derivatives, as well as their therapeutically acceptable salts, pharmaceutical preparations containing them and process for producing the active agent.

The compounds of general formula (I) are basic substances of pharmaceutical preparations of neurogenic and not neurogenic antiphlogistic, as well as paregoric effect, neuropathic hyperalgesia reducing ones, those for hindering rheumatic arthritis or treatment of destruction of bone or chondrus in joints, as well as for other diseases, which may be connected with other inflammatory processes e. g. asthma, eczema, psoriasis.

The compounds according to the general formula (I) 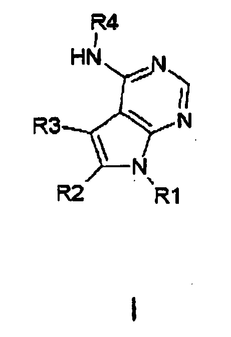 are new.

Their characteristic types may be described by general formulae (Ia), (Ib) and (Ic).

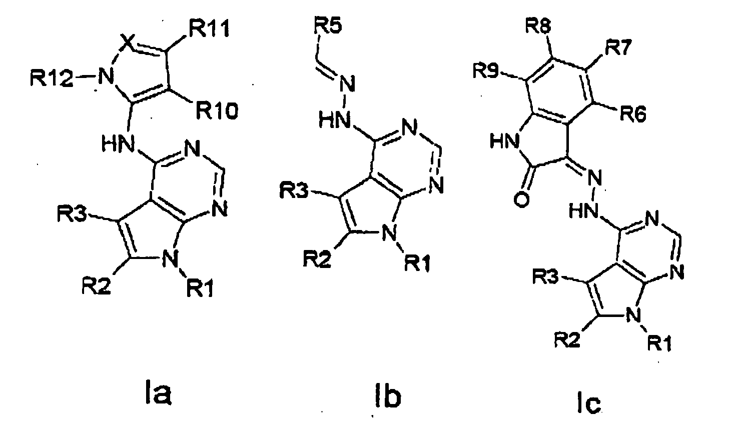

The compounds of 7H-pyrrolo[2,3-d]pyrimidine derivatives other than the above mentioned ones are known in the special literature as quinase inhibitors and antitumor agents acting in this way.

An important structural characteristic of the known active quinase inhibiting 7H-pyrrolo[2,3-d]pyrimidine derivatives is that only one from among the nitrogen atoms the one of pyrrhol in the position 7 and the other of the amine function connected to the fourth position may carry substituent (J. Med. Chem. 39 (1996) 2285-2292,Bioorg. Med. Chem. Letters 11(2001) 849-852).

In the case of compounds according to invention both of the above indicated places carry substituents.

Thus, the invention relates to 7H-pyrrolo[2,3-d]pyrimidine derivatives of the general formula (I), and to their therapeutically acceptable salts.

In the general formula (I) 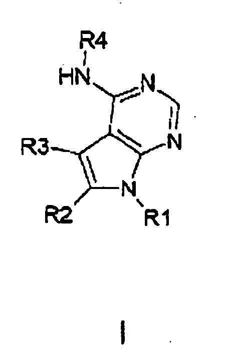
R1 is alkyl, aryl, heteroaryl or aryl-alkyl with 1-4 carbon atoms group, heteroaryl-alkyl with 1-4 carbon atoms group, motpholino-alkyl with 1-4 carbon atoms group or dialkylamino-alkyl with 1-4 carbon atoms group.
R2, R3 independently of each other are hydrogen, methyl, ethyl, propyl, isopropyl or cyclopropyl groups or R2 and R3 together are tetramethylene group.
R4 is 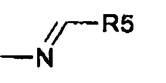 or 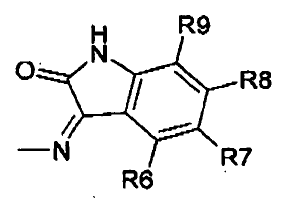 or 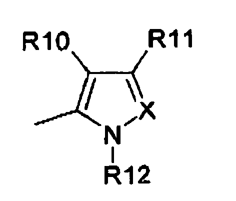 group,
   wherein:
   - R5: is substituted or unsubstituted aromatic or heteroaromatic ring where
   - R6, R7, R8 and R9: are independently hydrogen, halogen, nitro, amino, alkylamino, dialkylamino, hydroxy, methoxy, ethoxy, isopropoxy or sulfonyl group,
   - R10: is hydrogen or nitrile group,
   - R11: is hydrogen, methyl, ethyl, propyl, isopropyl, tert.- butyl group or tetramethylene ring connected to X
   - R12: is alkyl, aryl, heteroaryl, aryl-alkyl with 1-4 carbon atoms, heteroaryl-alkyl with 1-4 carbon atoms, morpholino-alkyl with 1-4 carbon atoms or dialkylamino-alkil with 1-4 carbon atoms group,
   - X: is carbon if R11 is a tetramethylene ring connected to X, otherwise nitrogen, methine, methyl-methine ethyl-methine, propyl-methine, isopropyl-methine, cyclopropyil-methine, tert.- butyl-methine or phenyl-methine group.

The therapeutically acceptable salts are advantageously acid-additive ones.

The most characteristic compounds of the general formula (I) may be defined by general formula (Ia), (Ib) and (Ic) where R1, R2, R3, R5, R6, R7, R8, R9, R10, R11, R12 and X are the same as mentioned above.

The invention relates also to a pharmaceutical preparation containing the compound of general formula (I) as active substance and therapeutically acceptable additives.

The pharmaceutical preparation is advantageously antiphlogistic and analgetic one, a preparation reducing neuropathic hyperalgesia and rheumatic arthritis, a preparation for hindering destruction of bones or chondrus, being applicable for treatment of other diseases, which may be connected with other inflammatory processes e. g. asthma, eczema or psoriasis.

The invention relates furthermore to a process for producing 7H-pyrrolo[2,3-d]pyrimidine derivatives of the general formula (I).

The process may be characterized in that a compound of general formula (II) 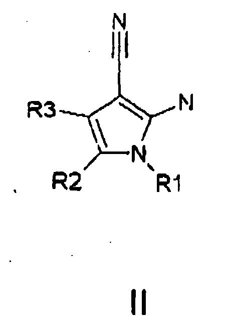 is produced from acetoine with amine and malonic acid dinitrile of molar equivalent quantities where R1, R2 and R3 are the same as mentioned above.

Formic acid of mass excess of 5 to 10 times is added, the mixture is mixed at reflux temperature during 1 hour to 2 days then the mixture is poured into icy water. The precipitated product is separated, dried then brought into reaction with phosphorus oxychloride of 5 to 10 times as much as the product at reflux temperature during 0.5 to 4 hours then the mixture is poured onto ice and the precipitated imidoyl chloride of general formula (III) 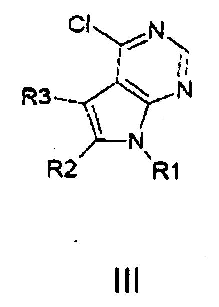 where R1, R2 and R3 are the same as mentioned above is separated, dried and evaporated then
A) The imidoyl chloride of general formula (III) produced in the above mentioned way is solved in an aprotic solvent and brought into reaction with amine of general formula (II) or (IV) 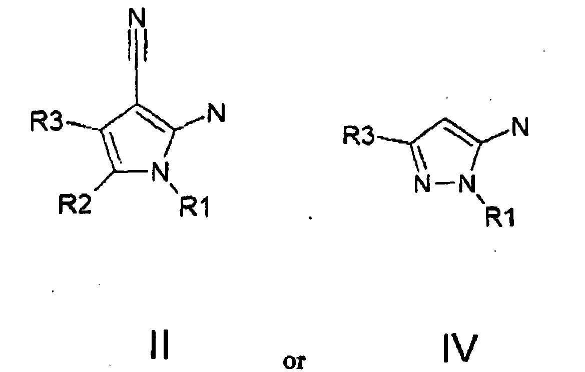 of equivalent quantity where R1 and R3 are the same as above in the formulas and NaH of 2 to 10 times of molar eqivalent excess is added. The reaction continues during 0.5 to 6 hours. The mixture is then poured onto ice and the precipitated product is separated and purified,
   or
B) the imidoyl chloride of general formula (III) produced in the above mentioned way is brought into reaction with hydrazine hydrate of 2 to 10 times of molar equivalent excess in a medium of polar organic solvent then the reaction product in the organic phase is separated, the organic phase is dried, evaporated then bruised with an apolar solvent. The hydrazine derivative produced in this way is mixed with a polar solvent and brought into reaction with aldehyde of equivalent quantity at 20 to 120 C° during 1 to 12 hours then the reaction product is separated,
   or
C) The hydrazine derivative produced according to the process described in B) is mixed with a polar organic solvent, brought into reaction with isatin of equivalent quantity then the reaction product is separated.

The compound of general formula (II) is produced in such a way that acetoine is brought into reaction with amine and malonic acid dinitrile of molar equivalent quantities. The compounds of general formula (II) are produced in this way. The reaction can be carried out in two stages in the same reaction mixture using either acidic or alkaline catalyst.

In the first stage the acetoine is brought into reaction with the suitable amine always with acidic catalyst (concentrated HCl or toluene sulphonic acid) in aprotic solvent (advantageously in toluene or benzene) at reflux temperature using water separating additive until the whole water produced in the reaction is removed then malonic acid dinitrile is added and the mixture is kept at reflux temperature until removal of water of equivalent quantity.

If amines of aniline type are used the solvent is changed to a protic one advantageously methanol, ethanol or isopropanol and they are brought into reaction with malonic acid dinitrile using alkaline medium (watery solution of KOH or NaOH) in inert atmosphere.

The intermediate compound of general formula (II) is filtered having cooled the mixture then mixed with formic acid of 5 to 10 mass excess (of 85 to 98 mass %) at reflux temperature during 1 hour to 2 days. The mixture is then poured onto icy water and the precipitated product is isolated either by filtering or shaking out with ethyl acetate at neutral pH.

The product is thoroughly dried in both cases then brought into reaction with phosphorus oxychloride of 5 to 10 times of mass excess at reflux temperature during 0.5 to 4 hours. The mixture containing phosphorus oxychloride is poured onto ice and having set pH to neutral the precipitated imidoyl chloride of general formula (III) is either filtered or shaken out with ethyl acetate, then the combined organic phase is dried and evaporated. The imidoyl chloride of general formula (III) received after filtering or shaking out and evaporation is solved in an aprotic solvent (THF, dioxane, DMSO or DMF) or in the mixture of them.

In the version A) of the process the imidoyl chloride of the general formula (III) is brought into reaction with amine of general structure (II) or (IV) of equivalent quantity adding NaH of molar equivalent excess of 2 to 10 times during 0.5 to 6 hours. The reaction mixture is poured onto icy water and processed, the precipitated product is filtered and the compounds of general formula (Ia) are produced by crystallizing from dioxane ethylacetate hexane mixture.

In the version B) of the process the imidoyl chloride of the general formula (III) produced in the above described way is brought into reaction with hydrazine hydrate of molar equivalent excess of 2 to 10 in a polar organic solvent (advantageously in methanol, ethanol, isopropanol, DMF, DMSO or in a mixture of any proportion of these solvents) then the solvent is extracted under reduced pressure and thereafter the product received in this way is separated between ethyl acetate and water.

The organic phase is evaporated after drying and the hydrazine derivative is received by bruising the organic phase with an apolar solvent (advantageously with hexane or ether), which is brought into reaction with aldehydes in a polar organic solvent (advantageously in methanol, ethanol, isopropanol, DMF, DMSO solvent or in glacial acetic acid) at a temperature of 20 to 100 C° during 1 to 12 hours. If the solvent is adequately chosen the product precipitates and may be separated by filtering after cooling of the mixture.

The compounds of the general formula (Ib) are obtained after a washing with an apolar solvent (advantageously with ether or hexane).

The version C) of the process is performed similarly but the hydrazine derivative is brought into reaction with isatin.

The compounds of general formula (Ic) are produced in this way.

The intermediate compounds used in the process according to the invention are partly known as intermediate products of the above mentioned quinase inhibitor compounds, such as im2_1 to im3_3, im3_5 and im4_1 to 4, they are partly new such as the compounds given in the examples 3, 5, 6, and 8 to 11 (see Table 1).

The essence of the invention is based also on the recognition that new structures may be created by linking together the reactive intermediate products of known biologically active compounds using the process described in the Example 4 and compounds of new structures are obtained having astoundingly different effects from those of the known compounds.

The essence of the process given in the Example 4 is that amines of general formulas (II) and (IV) solved in anhydrous dimethyl sulfoxide can be transformed into sodium salts by adding sodium hydride and the salts can be combined with imidoyl chlorides characterized by the general formula (III) at room temperature, and compounds of general structure (Ia) may be obtained in this way.

Connection of imidoyl chlorides and heterocyclic amines in presence of sodium hydride, as well as the received pyrazolyl amino and pyrrolyl amino derivatives are unknown in the literature.

Compounds having the same biological effect may be obtained also in the way that imidoyl chlorides characterized by the general formula (III) are brought into reaction first with hydrazine hydrate in a medium containing ethanol and the 4-hydrazino-7H-pyrrolo-[2,3-d]pyrimidine derivatives obtained in this way are brought into reaction with aldehydes or isatins as described in the Examples 7 and 12 to 15 and compounds of general formulas (Ib) or (Ic) are obtained in this way.

Condensation reactions of benzaldehydes and isatins in ethanol containing medium are well-known in the literature but from among the hydrazones obtainable from 4-hydrazino-7H-pyrrolo[2,3-d]pyrimidine derivatives only those having phenyl substituent in the position 5 are described up to now (J. Chem. Res. Miniprint 12 (1997) 2771-2789).

These known 5-phenyl derivatives proved to be completely ineffective in the models of inflammations described in the invention.

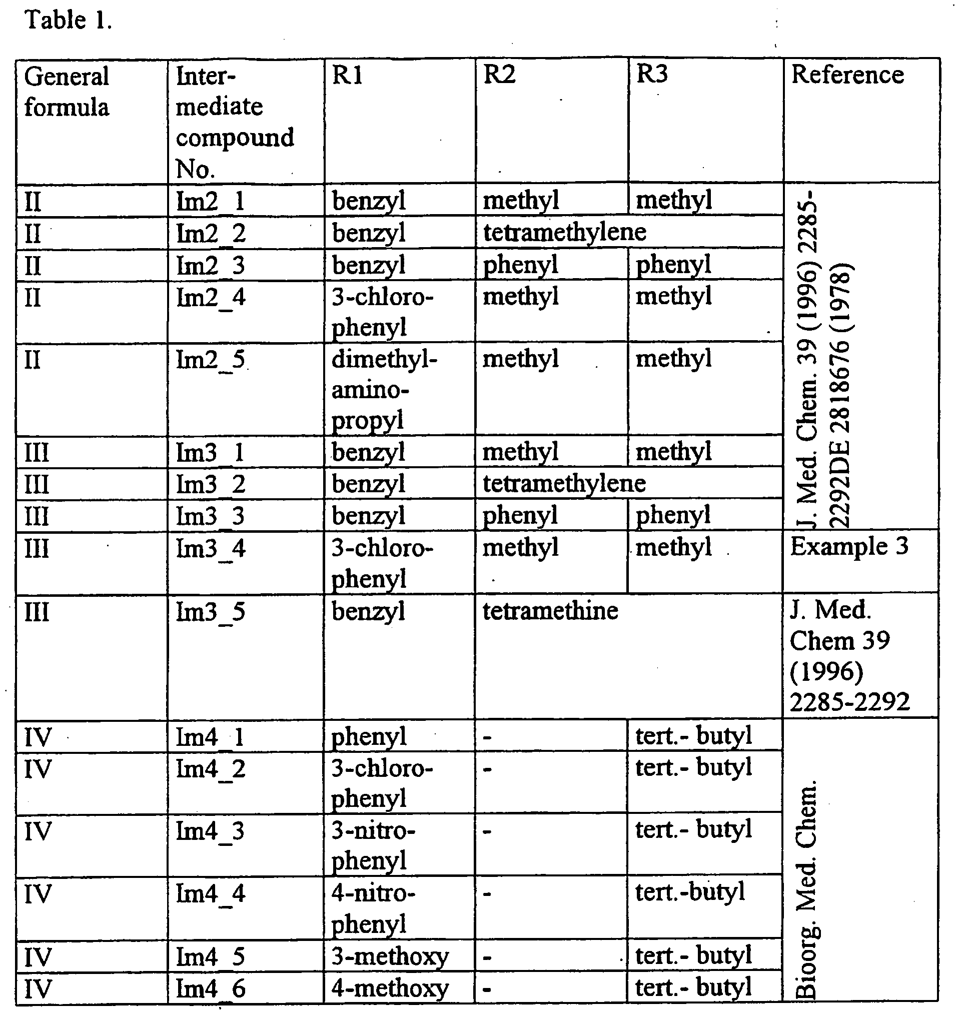

The compounds according to the invention are analgesic and antiphlogistic ones, which may be potentially orally administered.

An opportunity emerged to hinder neurogenic inflammation wherein the sensory neuronopeptides such as substance P (SP) or calcitonine gene related peptide (CGRP) released from primary afferents have important role, as well as to discover an analgesic of new type on the base of efficiency examination of heptapeptide somatostatine analogue TT-232.

We found out on the base of our previous examinations that that the TT-232 is a potent analgesic hindering both the neurogenic and non-neurogenic inflammation, which is effective also in the neuropathic nociceptive model. As the TT-232 is a peptide structure and cannot be orally absorbed, we began simultaneously with the clinical trials examinations of the compound to examine its peptidomimetic analogues to create a compound, which may be orally absorbed and has a wide spectrum of analgetic and anti-inflammatory effects similarly to the TT-232.

The in vitro tests were performed with electric excitation of sensoric neuron elements of an isolated rat trachea. The mediator of neurogenic inflammation the P-substance is released under influence of excitation from the capsaicine sensitive neuron elements and the concentration of P-substance in the water bath may be measured by radio immunoassay. Somatostatine antagonists, as well as the TT-232 hinder the excitation of terminal nerves so the quantity of released neuropeptide becomes less in the water bath.

The antiphlogistic efficiency of TT-232 against neurogenic and non-neurogenic inflammations was formerly tested primarily on rats (such as plasm extravasation provoked by mustard-oil, dextrane oedema, chronic arthritic oedema provoked by Freund adjuvant, plasm extravasation in articulations provoked by bradyquinine, carrageenine oedema, accumulation of leucocytes under influence of carrageenine). The oedema was provoked by alcoholic solution of capsaicine onto the mouse ear in the biological tests mentioned in the patent specification, and it was quantitatively evaluated by measurement of the mass of the ear and that of Evans's blue accumulation.

The antiphlogistic effect of the TT-232 against chronic inflammation provoked by Freund's adjuvant was demonstrated formerly on Wistar's female rat. This is the experimental model of rheumatoid arthritis. The phase II clinical trials of TT-232 on patients suffering from rheumatoid arthritis was performed in 2004. Wee took into consideration that the Freund's adjuvant aggravates symptoms implying considerable deformation of articulations on rats of Lewis strain, so the effect of TT-232 was examined in addition to the swelling of articulation provoked by Freund's adjuvant also to the destruction of bone to the infiltration of synovium lymphocytes and leucocytes and to the development of mechanical hyperalgesia, as well.

The following apparatuses were used in the experiments described in the patent specification.

The NMR photos were taken by apparatus Bruker AC300.

The apparatus used for measurement of HPLC was:
Waters HPLC with a detector ZMD MS and an UV one Waters 996 DAD provided with a column Supelco Discovery RP-Amide C16 wherein the gradient of acetonitrile is 10 to 1-00 % (of formic acid content of 0.05 %) per 6 minutes, the flowing rate is 3 ml per minute.

The melting points were measured by the apparatus Büchi Melting Point B-540.

The compounds according to the invention, the process for producing them and experiments to determine their effects are presented in the examples below.

### Example 1

### Process for producing 2-amino-4,5-dimethyl-1-(3-chlorophenyl)-1H-pyrrolo-3-carbonitrile

18 g (0.2 moles) of acetoine and 21 ml (0.2 moles) of 3-chloroaniline were solved in 300 ml of toluene then 0.2 ml of concentrated HCl was added. The reaction mixture was refluxed for two and half hours then evaporated to the half of volume at atmospheric pressure finally the whole solvent quantity was distilled in vacuum. The received material was solved in 350 ml of ethanol first 13.2 ml (0.2 moles) of malonic acid dinitrile was added then 11.2 g of KOH solved in 50 ml of water was dropped into it and cooled with ice. The mixture was refluxed for two hours then let to cool. The received crystalline product was filtered and washed with hexane (45.2 g; 92%).

### Example 2

### Process for producing 5,6-dimethyl-7-(3-chlorophenyl)-7H-pyrrolo[2,3-d]pyrimidine-4-ole

40 g of 2-amino-4,5-dimethyl-1-(3-chlorophenyl)amino-3-cyano-pyrrole (Example 1) was refluxed in 200 g of formic acid for 10 hours. The mixture was then poured onto ice and stirred for 1 hour. The precipitated material was filtered out and washed with water. The product was dried at room temperature and used in the following reaction without further purification. (27 g; 60 %).

### Example 3

### Process for producing 5,6-dimethyl-4-chloro-7-(3-chlorophenyl)-7H-pyrrolo[2,3--d]pyrimidine

26.9 g of 4-hydroxy-5,6-dimethyl-7-((3-chlorophenyl)amino) pyrrolo[2,3--d]pyrimidine (Example 2) was solved in 125 ml of POCl₃ and the mixture was refluxed for 1 hour, then poured onto ice-water of great excess of at least ten times greater volume, then the precipitated product was filtered off after complete decomposition of POCl₃ then washed with water and dried. (26.28 g; 92 %).

### Example 4

### Process for producing [5-tert-butyl-2-(3-nitro-phenyl)-2H-pyrazole-3-yl]-[7-(3-chlorophenyl)-5,6-dimethyl-7H-pyrrolo[2,3-d]pyrimidine-4-yl]-amifie.

0.292 g (1 millimole) of 5,6-dimethyl-4-chloro-7-((3-chlorophenyl)amino) pyrrolo[2,3-d]pyrimidine (Example 3) and 0.26 g (1 millimole) of 5-tert.--butyl-2-(3-nitro-phenyl)-2H-pyrazole-3-yl-amine (im4_3) were solved in 4 ml of dimethyl sulfoxide and 200 mg of sodium hydride was added in several portions under strong mixing then the mixture was mixed at room temperature for 1 hour. The reaction mixture was processed by pouring onto ice then the precipitated deposit was filtered off and recrystallized in the mixture of ethyl acetate and hexane (0.31 g; 60 %).

### Example 5

### Process for producing [7-(3-chlorophenyl)-5,6-dimethyl-7H-pyrrolo[2,3-d]pyrimidine-4-yl]-hydrazine

2.92 g (10 millimoles) of 4-chloro-7-(3-chlorophenyl)-5,6-dimethyl-7H-pyrrolo[2,3-d]pyrimidine (Example 3) was boiled together with 3 ml of hydrazine hydrate in 30 ml of ethanol for 3 hours then the solvent and the excess of hydrazine hydrate was distilled at a reduced pressure. The residue of the evaporation was separated between water and ethyl acetate. The organic phase was evaporated after drying. The residue of evaporation was bruised with ether and [7-*(3-chlorophenyl)-5,6-dimethyl-7H-pyrrolo[2,3-d]pyrimidine-4-yl]hydrazine* was obtained in this way (1.9 g; 66 %).

### Example 6

### Process for producing [7-benzyl-5,6-dimethyl-7H-pyrrolo[2,3-d]pyrimidine-4-yl]-hydrazine.

The compound was produced as described in the Example 5 with the difference that the primary substance was the known intermediate product marked as im3_1 (2.71 g of the substance).
(1.92 g; 74 %)

### Example 7

### Process for producing 4-{[5,6-Dimethyl-7-(3-chlorophenyl)-7H-pyrrolo[2,3--d]pyrimidine-4-yl]-hydrazono-methyl}-benzene-1,2-diole.

0.287 g (1 millimole) of [7-(3-chlorophenyl)-5,6-dimethyl-7H-pyrrolo[2,3--d]pyrimidine-4-yl]-hydrazine (Example 5) and 0.138 g (1 millimole) of 3,4-dihydroxy-benzaldehyde were boiled in 4 ml of ethanol for 6 hours and the precipitated product i. e. 4-{[7-(3-chlorophenyl)-5,6-dimethyl-7H-pyrrolo[2,3-d]pyrimidine-4-yl]-hydrazonomethyl}-benzene-1,2-diole was filtered and washed with ether. (0.29g; 68 %).

### Example 8

### Process for producing 2-amino-4,5-dimethyl-1-((3-morpholine-4-yl-propyl)-1H pyrrol-3-carbonitrile.

8.8 g (0.1 moles) of acetoine, 2-hydroxy-3-butanone and 14.4 ml (0.1 moles) of 4-(3-aminopropyl) morpholine were solved in 170 ml of toluene in an apparatus containing water separator attachment then 0.1 ml of concentrated hydrochloric acid was added, the mixture was then refluxed, until it boiled at 110C° then let to cool for a short time then 6.6 ml of malonitrile was added. The mixture was then refluxed until it boiled at 110 C° then filtered through Cellit then cooled. The precipitated crystals were filtered (17.31 g; 66 %).

### Example 9

### Process for producing 5,6-Dimethyl-7-(3-morpholine-4-yl-propyl)-7H-pyrrolo[2,3--d]pyrimidine-4-ol.

3.761 g (14.33 millimoles) of 2-amino-4,5-dimethyl-1-(3-morpholine-4-yl-propyl)-1H-pyrrolo-3-carbonitrile) (Example 8) was refluxed in 38 ml of formic acid for I night. The mixture was diluted with water during the process and neutralized with NaHCO3 shaken out with ethyl acetate, then the organic phase was evaporated, the precipitated crystals were processed with hexane then filtered (3.265 g; 78.5 %).

### Example 10

### Process for producing 5,6-dimethyl-4-chloro-7-(3-morpholine-4-yl-propyl)-7H-pyrrolo[2,3-d]pyrimidine.

3 g (10.33 millimoles) of 5,6-dimethyl-7-(3-morpholine-4-yl-propyl)-7H-pyrrolo[2,3-d]pyrimidine-4-ole (Example 9) was refluxed in 15 ml of POCl3 for an hour. The mixture was then poured onto ice, neutralized with NaHCO₃, shaken out with ethyl acetate and evaporated. A brown oil was obtained (2.71 g; 85 %).

### Example 11

### Process for producing [5,6-dimethyl-7-(3-morpholine-4-yl-propyl)-7H-pyrrolo[2,3--d]pyrimidine-4-yl]-hydrazine.

1.785 g (5.78 millimoles) of 5,6-dimethyl-4-chloro-7-(3-morpholine-4-yl-propyl)-7H-pyrrolo[2,3-d]pyrimidine (Example 10)was solved in so much of ethanol just enough for solution (120 ml) then 17.8 ml of hydrazine hydrate was added. The mixture was refluxed for about 15 minutes then evaporated and separated between water and ethyl acetate. The organic phase was dried and evaporated. The crystals precipitated during evaporation were bruised with hexane then filtered (1.07 g; 61 %).

### Example 12

### Process for producing 4-{[5,6-dimethyl-7-(3-morpholine-1-yl-propyl)-7H-pyrrolo[2,3-d]pyrimidine-4-yl]-hydrazonomethyl}-katechole.

304.4 mg (1 millimole) of [5,6-dimethyl-7-(3-morpholine-4-yl-propyl)-7H-pyrrolo-[2,3-d]pyrimidine-4-yl]-hydrazine (Example 11) and 138.1 mg (1 millimole) of 3,4-dihydroxy-benzaldehyde were solved in 6 ml of ethanol then refluxed for half an hour. The precipitated crystals were filtered out then washed with ethanol and hexane (45 %).

### Example 13

### Process for producing 4-{[5,6-dimethyl-7-(3-morpholine-4-yl-propyl)-7H-pyrrolo[2,3-d]pyrimidine-4-yl]hydrazonomethyl}-2-nitro-phenol.

304 mg (1millimole) of [5,6-dimethyl-7-(3-morpholine-4-yl-propyl)-7H-pyrrolo-[2,3-d]pyrimidine-4-yl-hydrazine (Example 11) and 169 mg (1 millimole) of 3-nitro-4-hydroxy-benzaldehyde were refluxed in 6 ml of ethanol for 4 hours then let to cool then filtered and washed in ethanol and hexane (176 mg; 36 %).

### Example 14

### Process for producing 4-{[5,6-dimethyl-7-(3-morpholine-4-yl-propyl)-7H-pyrrolo[2,3-d]pyrimidine-4-yl]-hydrazonomethyl}-2-methoxy-phenol.

304 mg (1 millimole) of [5,6-dimethyl-7-(3-morpholine-4-yl-propyl)-7H-pyrrolo[2,3-d]pyrimidine-4-yl]-hydrazine (Example 11) with 152 mg (1 millimole) of vanillin were refluxed in 6 ml of ethanol the precipitated crystals were filtered out then washed with ethanol and hexane (221.37 mg; 50 %).

### Example 15

### Process for producing 4,6-dichloro-3-{[5,6-dimethyl-7-(3-morpholine-4-yl-propyl)-7H-pyrrolo-[2,3-d]pyrimidine-1-yl]-hydrazono}-1,3-dihydro-indole-2-on.

108 mg (0.5 millimole) of 4,6-dichloro-isatin was added to 152.2 mg (0.5 millimole) of [5,6-dimethyl-7-(3-morpholine-4-yl-propyl)-7H-pyrrolo[2,3-d]pyrimidine-4-yl]-hydrazine (Example 11) and the mixture was refluxed in 4 ml of ethanol. The precipitated orange yellow crystals were filtered then washed with ethanol and hexane (102 mg; 40 %).

### Example 16

### Process for producing [5-tert.--butyl-2-(3-nitro-phenyl)-2H-pyrazole-3-yl]-[5,6-dimethyl-7-(3-morpholine-4-yl-propyl)-7H-pyrrolo[2,3-d]pyrimidine-4-yl]-amine.

650 mg (2.5 millimoles) of 5-tert.--butyl-2-(3-nitro-phenyl)-2H-pyrazole-3-yl-amine and 772 mg (2.5 millimoles) of 5,6-dimethyl-4-chloro-7-(3-morpholine-4-yl-propyl)-7H-pyrrolo[2,3-d]pyrimidine (Example 10) were solved in 4 ml of DMSO then 200 mg of NaH was added, and the mixture was stirred at room temperature for half an hour then the mixture was poured onto ice. The mixture was extracted with EtOAc dried and evaporated. The received brown oil was separated in a column, evaporated then bruised with hexane, the precipitated yellow crystals were filtered (272.2 mg; 20 %).

### Examples 17 to 91

The formulas of the compounds according to examples and the methods of production are given in the Table 2, the physical and chemical characteristics of the compounds are given in the Table 3.

### Example 92

### Process for producing [5-tert.--Butyl-2-(4-methoxy-phenyl)-2H-pyrazole-3-yl]-[5,6-dimethyl-7-(6-methyl-pyridine-2-yl)-7H-pyrrolo[2,3-d]pyrimidine-4-yl]-amine hydrochloride.

48 mg of the compound according to Example 91 was solved in I ml of anhydrous dioxane and 0.2 ml of 4M hydrochloric acid in ether was added to the mixture. The precipitated yellow deposit was filtered and washed with ether (36 mg; 70 %), melting point is 97 to 98 C°.

**Table 2.**

| Example No | Chemical name | Formula | M. w. | Used preparation process (Example No) | Used intermediate products (Example No) | Used commercial and literary starting materials | Yield (%) |
|---|---|---|---|---|---|---|---|
| 1 | 2-amino-1-4,5-dimethyl-(3-chlorophenyl)-1H-pyrrolo-3-carbonitrile | C13H12ClN3 | 245.7137 | 1 | | malonitrile, acetoine and 3-chloro-aniline | 92 |
| 2 | 5,6-dimethyl-7-(3-chlorophenyl)-7H-pyrrolo[2,3-d]pyrimidin-4-ol | C14H12ClN3O | 273.7242 | 2 | 1 | | 60 |
| 3 | 5,6-dimethyl-4-chloro-7-(3-chlorophenyl)-7H-pyrrolo[2,3-d]pyrimidine | C14H11Cl2N3 | 292.1699 | 3 | 2 | | 92 |
| 4 | [5-terc-buthyl-2-(3-nitro-phenyl)-2H-pyrazole-3-yl]-[5,6-dimethyl-7-(3-chlorophenyl)-7H-pyrrolo[2,3-d]pyrimidine-4-yl]-amine | C27H26ClN7O2 | 516.007 | 4 | | m3_4 és im4_3 | 60 |
| 5 | [5,6-dimethyl-7-(3-chlorophenyl)-7H-pyrrolo[2,3-d]pyrimidine-4-yl]-hydrazine | C14H14ClN5 | 287.7542 | 5 | 3 | | 66 |
| 6 | (7-benzyl-5,6-dimethyl-7H-pyrrolo[2,3-d]pyrimidine-4-yl)-hydrazine | C15H17N5 | 267.3362 | 6 | | m3_1 | 74 |
| 7 | 4-{[5,6-dimethyl-7-(3-chlorophenyl)-7H-pyrrolo[2,3-d]pyrimidine-4-yl]-hydrazonomethyl}-pyrokatechine | C21H18ClN5O2 | 407.8629 | 7 | 5 | 3,4-dihydroxy-benzaldehyde | 68 |
| 8 | 2-amineo-4,5-dimethyl-1-(3-morpholine-4-yl-propyl)-1H-pyrrol-3-carbonitrile | C14H22N4O | 262.3576 | 8 | | | 66 |
| 9 | 5,6-dimethyl-7-(3-morpholine-4-yl-propyl)-7H-pyrrolo[2,3-d]pyrimidine-4-ol | C15H22N4O2 | 290.3682 | 9 | | | 79 |
| 10 | 5,6-dimethyl-4-chloro-7-(3-morpholine-4-yl-propyl)-7H-pyrrolo[2,3-d]pyrimidine | C15H21ClN4O | 308.8138 | 10 | 9 | | 85 |
| 11 | [5,6-dimethyl-7-(3-morpholine-4-yl-propyl)-7H-pyrrolo[2,3-d]pyrimidine-4-yl]-hydrazine | C15H24N6O | 304.3981 | 11 | 10 | | 61 |
| 12 | 4-{[5,6-dimethyl-7-(3-morpholine-4-yl-propyl)-7H-pyrrolo[2,3-d]pyrimidine-4-yl]-hydrazonomethyl}pyrokatechine | C22H28N6O3 | 424.5069 | 12 | | 3,4-dihydroxy-benzaldehyde | 45 |
| 13 | 4-{[5,6-dimethyl-7-(3-morpholine-4-yl-propyl)-7H-pyrrolo[2,3-d]pyrimidine-4-yl]-hydrazonomethyl}-2-nitro-phenol | C22H27N7O4 | 453.505 | 13 | 11 | 4-hydroxy-3-nitro-benzaldehyde | 36 |
| 14 | 4-{[5,6-dimethyl-7-(3-morpholine-4-yl-propyl)-7H-pyrrolo[2,3-d]pyrimidine-4-yl]-hydrazonomethyl}-2-methoxy-phenol | C23H30N6O3 | 438.534 | 14 | 11 | 4-hydroxy-3-methoxy-benzaldehyde | 50 |
| 15 | 4,6-dichloro-3-{[5,6-dimethyl-7-(3-morpholine-4-yl-propyl)-7H-pyrrolo[2,3-d]pyrimidine-4-yl]-hydrazono}-1,3-dihydro-indole-2-on | C23H25Cl2N7O2 | 502.4074 15 | | 11 | 4,6-dichloroisatin | 41 |
| 16 | [5-tert-butyl-2-(3-nitro-phenyl)-2H-pyrazole-3-yl]-[5,6-dimethyl-7-(3-morpholine-4-yl-propyl)-7H-pyrrolo[2,3-d]pyrimidine-4-yl]-amine | C28H36N8O3 | 532.6509 | 16 | 10 | im4_3 | 20 |
| 17 | N-(7-benzyl-5,6-dimethyl-7H-pyrrolo[2,3-d]pyrimidine-4-yl)-N'-(2-methoxy-benzylidene)-hydrazine | C23H23N5O | 385.4727 | 7 | 6 | 2-methoxy-benzaldehyde | 54 |
| 18 | 4-[(7-benzyl-5,6-dimethyl-7H-pyrrolo[2,3-d]pyrimidine-4-yl)-hydrazonomethyl]-pyrokatechine | C22H21N5O2 | 387.445 | 7 | 6 | 3,4-dihydroxy-benzaldehyde | 62 |
| 19 | N-[5,6-dimethyl-7-(3-chlorophenyl)-7H-pyrrolo[2,3-d]pyrimidine-4-yl]-N'-naphthalene-1-yl-methylene-hydrazine | C25H2OClN5 | 425.9247 | 7 | 5 | naphtyl-1-carbaldehyde | 35 |
| 20 | N-[5,6-dimethyl-7-(3-chlorophenyl)-7H-pyrrolo[2,3--d]pyrimidine-4-yl]-N'-(4-trifluoro-methyl-benzylidene)-hydrazine | C22H17ClF3N5 | 443.8625 | 7 | 5 | 4-trifluoromethyl-benzaldehyde | 62 |
| 21 | 4-{[5,6-dimethyl-7-(3-chlorophenyl)-7H-pyrrolo[2,3-d]pyrimidine-4-yl]-hydrazonomethyl}-2-nitro-phenol | C21H17ClN6O3 | 436.861 | 7 | 5 | 4-hydroxy-3-nitro-benzaldehyde | 33 |
| 22 | N-(3,4-Bis-benzyloxi- benzylidene)-N'-[5,6-dimethyl-7-(3-chlorophenyl)-7H-pyrrolo[2,3 -d]pyrimidine-4-yl]-hydrazine | C35H30ClN5O2 | 588.1147 | 7 | 5 | 3,4-dibenzyloxi-benzaldehyde | 58 |
| 23 | N-[5,6-dimethyl-7-(3-chlorophenyl)-7H-pyrrolo[2,3-d]pyrimidine-4-yl]-N'-(3-nitro- benzylidene)-hydrazine | C21H17ClN6O2 | 420.8616 | 7 | 5 | 3-nitro-benzaldehyde | 72 |
| 24 | 4-{[5,6-dimethyl-7-(3-chlorophenyl)-7H-pyrrolo[2,3- d]pyrimidine-4-yl]-hydrazonomethyl}-2-methoxy- phenol | C22H20ClN5O2 | 421.89 | 7 | 5 | 4-hydroxy-3-methoxy-benzaldehyde | 35 |
| 25 | N-(7-benzyl-5,6-dimethyl-7H-pyrrolo[2,3-d]pylimidine-4-yl)-N'-naftalèn-1-ylmethylene-hydrazine | C26H23N5 | 405.5067 | 7 | 6 | naphtyl-1-carbaldehyde | 58 |
| 26 | N-(7-benzyl-5,6-dimethyl-7H-pyrrolo[2,3-d]pyrimidine-4-yl)-N'-(4-trifluoromethyl-benzylidene)-hydrazine | C23H20F3N5 | 423.4446 | 7 | 6 | 4-trifluoromethyl-benzaldehyde | 81 |
| 27 | N-(7-benzyl-5,6-dimethyl-7H-pyrrolo[2,3-d]pyrimidine-4-yl)-N'-thiophene-2-yl-methylene-hydrazine . | C20H19N5S | 361.4719 | 7 | 6 | thiophene-2 carbaldehyde | 57 |
| 28 | 4-[(7-benzyl-5,6-dimethyl-7H-pyrrolo[2,3-d]pyrimidine-4-yl)-hydrazonomethyl]-2-nitro-phenol | C22H20N6O3 | 416.4431 | 7 | 6 | 4-hydroxy-3-nitro-benzaldehyde | 95 |
| 29 | 4-[(7-benzyl-5,6-dimethyl-7H-pyrrolo[2,3-d]pyrimidine-4-yl)-hydrazonomethyl]-2,6-dimethyl-phenol | C24H25N50 | 399.4998 | 7 | 6 | 3,5-dimethyl-4-hydroxy-benzaldehyde | 91 |
| 30 | N-(7-benzyl-5,6-dimethyl-7H-pyrrolo[2,3-d]pyrimidine-4-yl)-N'-(3,4'bis-benzyloxi-benzylidene)-hydrazine | C36H33N5O2 | 567.6967 | 7 | 6 | 3,4-dibenzyloxy-benzaldehyde | 67 |
| 31 | {4-[(7-benzyl-5,6-dimethyl-7H-pyrrolo[2,3-d]pyrimidine-4-yl)-hydrazonomethyl]-phenyl}-dimethyl-amine | C24H26N6 | 398.515 | 7 | 6 | 4-dimethylamino-benzaldehyde | 21 |
| 32 | N-(7-benzyl-5,6-dimethyl-7H-pyrrolo[2,3-d]pyrimidine-4-yl)-N'-(3-nitro-benzylidene)-hydrazine | C22H20N6O2 | 400.4437 | 7 | 6 | 3-nitro-benzaldehyde | 25 |
| 33 | 4-[(7-benzyl-5,6-dimethyl-7H-pyrrolo[2,3-d]pyrimidine-4-yl)-hydrazonomethyl]-2-methoxy-phenol | C23H23N502 | 401.4721 | 7 | 6 | 4-hydroxy-3-methoxy-benzaldehyde | 20 |
| 34 | N-(7-benzyl-5,6-dimethyl-7H-pyrrolo[2,3-d]pyrimidine-4-yl)-N'-(4-izopropyl-benzylidene)-hydrazine | C25H27N5 | 397.5274 | 7 | 6 | 4-isopropyl-benzaldehyde | 58 |
| 35 | 4-[(7-benzyl-5,6-dimethyl-7H-pyrrolo[2,3-d]pyrimidine-4-yl)-hydrazonomethyl]-benzonitril | C23H20N6 | 380.4561 | 7 | 6 | 4-cyano-benzaldehyde | 69 |
| 36 | 4-{[5,6-dimethyl-7-(3-chlorophenyl)-7H-pyrrolo[2,3--d]pyrimidine-4-yl]- hydrazonomethyl}-2,6-dimethyl-phenol | C23H22ClN5O | 419.9177 | 7 | 5 | 3,5-dimethyl-4-hydroxy-benzaldehyde | 47 |
| 37 | 4-{[5,6-dimethyl-7-(3-chlorophenyl)-7H-pyrrolo[2,3-d]pyrimidine-4-yl]-hydrazonomethyl}-benzonitrile | C22H17ClN6 | 400.874 | 7 | 5 | 4-cyano-benzaldehyde | 62 |
| 38 | 5-bromo-3-{[5,6-dimethyl-7-(3-chlorophenyl)-7H-pyrrolo[2,3-d]pyrimidine-4-yl]-hydrazono}-1,3-dihydro-indole-2-on | C22H16BrClN60 | 495.7694 | 7 | 5 | 5-bromo-isatin | 50 |
| 39 | 3-{[5,6-dimethyl-7-(3-chlorophenyl)-7H-pyrrolo[2,3-d]pyrimidine-4-yl]-hydrazono)-1,3-dihydro-indole-2-on | C22H17ClN6O | 416.8734 | 7 | 5 | satin | 33 |
| 40 | 5-chloro-3-{[5,6-dimethyl-7-(3-chlorophenyl)-7H-pyrrolo[2,3-d]pyrimidine-4-yl]-hydrazono)-1,3-dihydro-indole-2-on | C22H16Cl2N6O | 451.3184 | 7 | 5 | 5-chloro-isatin | 63 |
| 41 | 4,7-dichloro-3-{[5,6-dimethyl-7-(3-chlorophenyl)-7H-pyrrolo[2,3-d]pyrimidine-4-yl]-hydrazono}-1,3-dihydro-indole-2-on | C22H15Cl3N6O | 485.7635 | 7 | 5 | 4,7-chloro-isatin | 78 |
| 42 | 3-{[5,6-dimethyl-7-(3-chlorophenyl)-7H-pyrrolo[2,3-d]pyrimidine-4-yl]-hydrazono}-5-methyl-1,3-dihydro-indole,2-on | C23H19ClN6O | 430.9005 | 7 | 5 | 5-methyl-isatin | 63 |
| 43 | 3-{[5,6-dimethyl-7-(3-chlorophenyl)-7H-pyrrolo[2,3-d]pyrimidine-4-yl]-hydrazono}-5-fluoro-1,3-dihydro-indole-2-on | C22H16ClFN6O | 434.8638 | 7 | 5 | 5-fluoro-isatin | 72 |
| 44 | 3-{[5,6-dimethyl-7-(3-chlorophenyl)-7H-pyrrolo[2,3-d]pyrimidine-4-yl]-hydrazono}-5-trifluoromethoxy-1,3-dihydro-indole-2-on | C23H16ClF3N6O2 | 500.8712 | 7 | 5 | 5-trifluoromethoxy-isatin | 69 |
| 45 | 3-[(7-benzyl-5,6-dimethyl-7H-pyrrolo[2,3-d]pyrimidine-4-yl)-hydrazono]-5-bromo-1,3-dihydro-indole-2-on | C23H19BrN6O | 475.3515 | 7 | 6 | 5-bromo-isatin | 46 |
| 46 | 3-[(7-benzyl-5,6-dimethyl-7H-pyrrolo[2,3-d]pyrimidine-4-yl)-hydrazono]-1,3-dihydro-indole-2-on | C23H20N60 | 396.4555 | 7 | 6 | isatin | 47 |
| 47 | 3-[(7-benzyl-5,6-dimethyl-7H-pyrrolo[2,3-d]pyrimidine-4-yl)-hydrazono]-2-oxo-2,3-dihydro-1H-indole-5-sulphonic acid | C23H20N6O4S | 476.5177 7 | | 6 | isatin-5-sulphonic acid | 15 |
| 48 | 3-[(7-benzyl-5,6-dimethyl-7H-pyrrolo[2,3-d]pyrimidine-4-yl)-hydrazono]-5-chloro-1,3-dihydro-indole-2-on | C23H19ClN6O | 430.9005 | 7 | 6 | 5-chloro-isatin | 55 |
| 49 | 3-[(7-benzyl-5,6-dimethyl-7H-pyrrolo[2,3-d]pyrimidine-4-yl)-hydrazono]-4,6-dichloro-1,3-dihydro-indole-2-on | C23H18Cl2N6O | 465.3455 | 7 | 6 | 4,6-dichloro-isatin | 81 |
| 50 | 3-[(7-benzyl-5,6-dimethyl-7H-pyrrolo[2,3-d]pyrimidine-4-yl)-hydrazono].4,7-dichloro,1,3. dihydro-indole-2-on | C23H18Cl2N6O | 465.3455 | 7 | 6 | 4,6-dichloro-isatin | 48 |
| 51 | (7-benzyl-5,6-dimethyl-7H-pyrrolo[2,3-d]pyrimidine-4-yl)-(5-terc-buthyl-2-phenyl-2H-pyrazole-3-yl)-amine | C28H30N6 | 450.5915 | 4 | | im3_1 és im4_1 | 33 |
| 52 | (7-benzyl-5,6-dimethyl-7H-pyrrolo[2,3-d]pyrimidine-4-yl)-[5-tert.-buthyl-2-(4-nitro-phenyl)-2H-pyrazole-3-yl]-amine | C28H29N7O2 | 495.589 | 4 | | im3_1 és im4_4 | 71 |
| 53 | (7-benzyl-5,6-dimethyl-7H-pyrrolo[2,3-d]pyrimidine-4-yl)-[5-tert-buthyl-2-(3-nitro-phenyl)-2H-pyrazole-3-yl]-amine | C28H29N7O2 | 495.589 | 4 | | im_3 1 és im4_3 | 72 |
| 54 | (7-benzyl-5,6-dimethyl-7H-pyrrolo[2,3-d]pyrimidine-4-yl)-[5-tert.-buthyl-2-(3-fluoro-phenyl)-2H-pyrazole-3-yl]-amine | C28H29FN6 | 468.5819 | 4 | | im3_1 és im4_2 | 81 |
| 55 | (5-tert.-buthyl-2-phenyl-2H-pyrazole-3-yl)-15,6-dimethyl-7-(3-chlorophenyl)-7H-pyrrolo[2,3-d]pyrimidine-4-yl]-amine | C27H27CIN6 | 471.0094 | 4 | | m3_4 és im4 _1 | 57 |
| 56 | [5-tert.-buthyl-2-(3-fluoro-phenyl)-2H-pyrazole-3-yl]-[5,6-dimethyl-7-(3-chlorophenyl)-7H-pyrrolo[2,3-d]pyrimidine-4-yl]-amine | C27H26ClFN6 | 488.9999 | 4 | | im3_4 és im4_2 | 95 |
| 57 | [5-tert.-buthyl-2-(4-nitro-phenyl)-2H-pyrazole-3-yl]-[5,6-dimethyl-7-(3-chlorophenyl)-7H-pyrrolo[2,3--d]pyrimidine-4-yl]-amine | C27H26ClN7O2 | 516.007 | 4 | | im3 4 és im4_4 | 91 |
| 58 | (9-benzyl-6,7,8,9-tetrahydro-5H-1,3,9-triaza-fluoren-4-yl)-[5.tert-buthyl-2-(3-fluoro-phenyl)-2H-pyrazole-3-yl]-amine | C30H31FN6 | 494.6202 | 4 | | im3_2 és im4_2 | 67 |
| 59 | (9-benzyl-6,7,8,9-tetrahydro-5H-1,3,9-triaza-fluoren-4-yl)-(5-tert.-buthyl-2-phenyl-2H-pyrazole-3-yl)-amine | C30H32N6 | 476.6297 | 4 | | im3_2 és im4_1 | 59 |
| 60 | 5,6-dimethyl-1-(3-chlorophenyl)-2-[7-(3-chlorophenyl)-7H-pyrrolo- [2,3-d]pyrimidine-4-yl-amino]-4,5-dimethyl-1H-pyrrol-3-carbonitrile | C27H22Cl2N6 | 501.4226 | 4 | | im2_4 és im3_4 | 69 |
| 61 | 1-benzyl-2-[7-(3-chlorophenyl)-5,6-dimethyl-7H-pyrrolo[2,3-d]pyrimidine-4-yl-amino]-4,5,6,7-tetrahidro-1H-indole-3-carbonitrile | C30H27ClN6 | 507.0429 | 4 | | im2_2 és im3_4 | 66 |
| 62 | 1-benzyl-2-[5,6-dimethyl-7-(3-chlorophenyl)-7H-pyrrolo[2,3-d]pyrimidine-4-yl-amino]-4,5-diphenyl-1H-pyrrol-3-carbonitrile | C38H29ClN6 | 605.148 | 4 | | im2_3 és im3_4 | 60 |
| 63 | 1-benzyl-2-[5.6-dimethyl-7-(3-chlorophenyl)-7H-pyrrolo[2,3-d]pyrimidine-4-yl-amino]-4,5-dimethyl-1H-pyrrol-3-carbonitrile | C28H25CIN6 | 4881.0047 | 4 | | im2_1 és im3_4 | 74 |
| 64 | 2-(9-benzyl-9H-1,3,9-triaza-fluorene-4-ylamino)-1-(3-chloropenyl)-4,5-dimethyl-1H-pyrrol-3-carbonitrile | C30H23CIN6 | 503.011 | 4 | | im2_4 és im3_5 | 69 |
| 65 | 1-benzyl-2-(9-benzyl-9H-1,3,9-triaza-fluorene-4-yl-amino)-4,5,6,7-tetrahidro-1H-indole-3-carbonitrile | C33H28N6 | 508.6313 | 4 | | im2_2 és im3_5 | 68 |
| 66 | 1-benzyl-2-(9-benzyl-9H-1,3,9-traza-fluorene-4-yl-amino)-4,5-diphenyl-1H-pyrrol-3-carbonitrile | C41H30N6 | 606.7365 | 4 | | im2_3 és im3_5 | 70 |
| 67 | 1-benzyl-2-(9-benzyl-9H-1,3,9-triaza-fluorene-4-yl-amino)-4,5- dimethyl-1H-pyrrol-3-carbonitrile | C31H26N6 | 482.5931 | 4 | | im2_1 és im3_5 | 73 |
| 68 | 2-(9-benzyl-6,7,8,9-tetrahidro-5H-1,3,9-triaza-fluorene-4-yl-amino)-4,5-dimethyl-1-(3-chlorophenyl)-1H-pyrrol-3-carbonitrile | C30H27ClN6 | 507.0429 | 4 | | im2_4 és im3_2 | 68 |
| 69 | 1-benzyl-2-(9-benzyl-6,7,8,9-tetrahidro-5H-1,3,9-triaza-fluorene-4-yl-amino)-4,5,6,7-tetrahidro-1H-indole-3-carbonitrile | C33H32N6 | 512.6632 | 4 | | im2_2 és im3_2 | 48 |
| 70 | 1-benzyl-2-(9-benzyl-6,7,8,9-tetrahidro-5H-1,3,9-triaza-fluorene-4-yi-amino)-4,5-diphenyl-1H-pyrrol-3-carbonitrile | C41H34N6 | 610.7683 | 4 | | im2_3 és im3_3 | 55 |
| 71 | 1-benzyl-2-(9-benzyl-6,7,8,9-tetrahldro-5H-1,3,9-triaza-fluorene-4-yl-amino)-4,5-dimethyl-1H-pyrrol-3-carbonitrile | C31H30N6 | 486.625 | 4 | | im2_1 és im3_2 | 81 |
| 72 | 2-(7-benzyl-5,6-dimethyl-7H-pyrrolo[2,3-d]pydmidine-4--yl-amino)-1-(3-chlorophenyl)-4,5-dimethyl-1H-pyrrol-3-carbonitrile | C28H25ClN6 | 481.0047 | 4 | | im2_4 és im3_1 | 91 |
| 733 | 1-benzyl2-(7-benzyl-5,6-dimethyl-7H-pynolo[2,3-d]pyrimidin-4-ylamineo)-4,5,6,7-tetrahydro-1H-indole-3-carbonitrile | C31H30N6 | 486.625 | 4 | | im2_2 ém3_1 | 67 |
| 74 | 1-benzyl-2-(7-benzyl-5,6-dimethyl-7H-pyrrolo[2,3-d]pyrimidine-4-yl-amino)-4,5-diphenyl-1H-pyrrol-3-carbonitrile | C39H32N6 | 584.7301 | 4 | | im2_3 és im3_1 | 81 |
| 75 | 1-benzyl-2-(7-benzyl-5,6-dimethyl-7H-pyrrolo[2,3-d]pylimidine-4-yl-amino)-4,5-dimethyl-1H-pyrrol-3-carbonitrile | C29H28N6 | 460.5867 | 4 | | im2_1 és im3_1 | 49 |

**Table 3. Physical-chemical characters**

| Example No | M. w. | LC t_{R} | MS [M+H]⁺ or [M-H]⁻ | NMR methode | NMR spectrum | Melting point (°C) |
|---|---|---|---|---|---|---|
| 1 | 245.71 | 3,17 | 246 | 1H NMR, 300 MHz, DMSO-d6 (ppm) : | 7.52 (d, 2 H), 7.38 (s, 1H), 7.23 (m, 1 H), 5.46 (s, 2 H), 1.91 (s, 3 H), 1.74 (s, 3 H) | 148-150 |
| 2 | 273.72 | 2,97 | 274 | 1H NMR, 300 MHz, DMSO-d6 (ppm) : | 11.83 (s, 1H), 7.71 (s, 1H), 7.55 (m, 3 H), 7.36 (m, 1H), 2.29 (s, 3 H), 2.07 (s, 3 H) | 288-291 |
| 3 | 292.17 | 3,32 | 292 | 1H NMR, 300 MHz, DMSO-d6 (ppm) : | 7.60 (m, 4 H), 7.44 (m, 1H), 2.43 (s, 3 H), 2.23 (s, 3 H) | 158-161 |
| 4 | 516.01 | 3.60 | 514 | 1H NMR, 300 MHz, DMSO-d6 (ppm) : | 8.54(s, 1H); 8.07(d, 1H), 7.95(s, 1H), 7.71-7.47(m, 5H), 7.36(d, 1H), 6.48(s, 1H). 2.40(s, 3H), 2.17(s, 3H), 1.34(s, 9H) | 189-190 |
| 5 | 287.75 | 2.52 | 288 | 1H NMR, 300 MHz, DMSO-d6 (ppm) : | 7.71 (m, 4 H), 7.52 (m, 1H), 7.20(m, 3H), 2.49 (s, 3 H), 2.27 (s, 3 H) | 169-171 |
| 6 | 267.34 | 2.32 | 268 | 1H NMR, 300 MHz, DMSO-d6 (ppm) : | 8.15(s, 1H), 7.77(s, 1H), 7.23(m, 3H), 7.01 (d, 2H), 5.35(s, 2H), 4.44(s, 2H), 2.29(s, 3H), 2.13(s, 3H) | 195-198 |
| 7 | 407.86 | 2.76 | 406 | 1H NMR, 300 MHz, DMSO-d6 (ppm) : | 11.2, 10.47 (bs, 1H), 9(bs; 2H), 8.17-6.76(m, 9H), 2.54, 2.36(s, 3H), 2.19, 2.09(s, 3H) | 105-107 |
| 8 | 262.36 | 1.91 | 262 | 1H NMR, 300 MHz, DMSO-d6. (ppm) : | 5.74 (s, 2 H), 3.62 (t, 2 H), 3.55 (m, 4 H), 2.30 (bs, 4 H), 2.17 (t. 2 H), 1.96 (s, 3 H), 1.83 (s, 3 H), 1.66 (m, 2 H) | 135-138 |
| 9 | 290.37 | 1,63 | 290 | 1H NMR, 300 MHz, DMSO-d6 (ppm) : | 11.67 (s, 1H), 7.72 (s, 1H), 4.05 (t, 2 H), 3.52 (m, 4 H), 2.23 (m, 12 H), 1.76 (m, 2 H) | 192,5-195,3 |
| 10 | 308.81 | | | 1H NMR, 300 MHz, DMSO-d6 (ppm): | 8.28(s, 1H), 4.11(t, 2H), 3.50(t, 4H), 2.25(m, 12H), 1.77(m, 2H) | olaj |
| 11 | 304.40 | 0,55 | 304 | 1H NMR, 300 MHz, DMSO-d6 (ppm): | 8.11 (s, 1H), 7.67 (s, 1H), 4.40 (s, 2 H), 4.09 (t, 2 H), 3.53 (m, 4 H), 2.23 (m, 12 H), 1.78 (m, 2 H) | 115-117 |
| 12 | 424.51 | 1,92 | 424 | 1H NMR, 300 MHz, DMSO-d6 (s, (ppm) : | 10.30, 9.05 (bs, 1H), 8.13, 8.06 1H), 7.63-6.74 (m, 4H), 4.16, 4.04 (t, 2H), 3.53 (bs, 4H), 2.49 (s, 3H), 2.34 (bs, 6H), 2.20 (s, 3H), 1.80 (m, 2 H) | 219,4-222,4 |
| 13 | 453.50 | 2,17 | 453 | 1H NMR, 300 MHz, DMSO-d6H), (ppm) : | 11.50 (bs, 1H), 8.24-7.80 (m, 4 7.13 (d, 1H), 4.10 (t, 2 H), 3.55 (bs, 6 H), 2.29 (s, 3 H), 2.27 (s, 3 H), 1.81 (m, 2 H) | 158,8-161,5 |
| 14 | 438.53 | 2,54 | 438 | 1H NMR, 300 MHz, DMSO-d6 (ppm) : | 10.45, 9.20 (bs 1H), 8.20-6.78 (m, 5 H), 4.16, 4.05 (t, 2 H), 3.86 (s, 3 H), 3.81 (s, 3 H), 3.55 (bs; 4 H), 2.46 (s, 3 H), 2.29 (bs, 6 H), 2.21 (s, 3 H), 1.18 (m, 2 H) | 201,3-205 |
| 15 | 502.41 | 2,78 | 501 | 1H NMR, 300 MHz, DMSO-d6 (ppm) : | 13.35 (bs, 1 H), 8.42 (s, 1 H), 7.17 (s, 1 H), 6.97 (s, 1 H), 4.25 (t, 2 H), 3.55 (bs, 4 H), 2.51, (bs, 6 H), 2.40 (s, 3 H), 2.31 (s, 3 H), 1.90 (m, 2 H) | 265,6-268,5 |
| 16 | 532.65 | 3.10 | 532 | 1H NMR, 300 MHz, DMSO-d63H), (ppm) : | 8.44(s, 1 H), 8.38(s, 1 H), 8.02(m, 7.63(t, 1H), 6.43(s, 1H). 4.09(t, 2H), 3.49(t, 4H), 2.31-2.18(m, 12H), 1.77(m, 2H), 1.32(s, 9H) | 120,9-125,3 |
| 17 | 385.47 | 2.84 | 386 | 1H NMR, 300 MHz, DMSO-d611H), (ppm) : | 11.61, 10.76(s, 1H), 8.60-6.97(m, 5.43, 5.31 (s, 2H), 3.86(s, 3H), 2.39, 2.31(s, 3H), 2.21, 2.10(s, 3H), | 214-216 |
| 18 | 387.44 | 2.73 | 386 | 1H NMR, 300 MHz, (ppm) : | 11.48, 10.63(s, 1 H), 9.39(bs, 2H), DMSO-d68.24-6.80(m, 10), 5.45, 530(s, 2H), 3.89, 3.83(s, 3H), 2.48, 2.30(s, 3H), 2.22, 2.10(s, 3H) | 257-259 |
| 19 | 425.92 | 2.97 | 426 | 1H NMR, 300 MHz, DMSO-d613H), (ppm) : | 11.72, 10.77(s, 1H), 9.13-7.40(m, 2.53, 2.42(s, 3H), 2.20, 2.13(s, 3H) | 133-140 |
| 20 | 443.86 | 2.98 | 442 | 1H NMR, 300 MHz, DMSO-d610H), (ppm) : | 11.89, 11.02(s, 1 H), 8.42-7.40(m, 2.45, 2.40(s, 3H), 2.20, 2.12(s, 3H) | 238-239 |
| 21 | 436.86 | 2.85 | 437 | 1H NMR, 300 MHz, DMSO-d610H), (ppm) : | 11.92, 11.03(s, 1H), 8.70-7.41 (m, 2.53, 2.39(s, 3H), 2.20-2.11 (s, 3H) | 204,2-206,5 |
| 22 | 588.11 | 3.07 | 586 | 1H NMR, 300 MHz, DMSO-d618H), (ppm) : | 11.60, 10.73(s, 1H), 8.27-7.12(m, 5.19(s, 4H), 2.43, 2.38(s, 3H), 2.19, 2.10(s, 3H) | 101-109 |
| 23 | 420.86 | 2.91 | 421 | 1H NMR, 300 MHz, DMSO-d610H), (ppm) : | 11.94, 11.05(s, 1H), 8.76-7.42(m, 2.53, 2.40(s, 3H), 2.21-2.12(s, 3H) | 218-219 |
| 24 | 421.89 | 2.77 | 420 | 1H NMR, 300 MHz, DMSO-d6 (ppm) : | 11.53, 10.65(s, 1H), 9.40(bs, 1H), 8.25-6.80(m, 9H), 3.86, 3.82(s,3H), 2.53, 2.50(s, 3H), 2.19,2.09(s, 3H) | 146-187 |
| 25 | 405.51 | 2.95 | 404 | 1H NMR, 300 MHz, DMSO-d6 (ppm) : | 11.66, 10.71 (s, 1H), 9.11-7.07(m, 14H), 5.45, 5..33(s, 2H), 2.43, 239(s, 3H), 2.22, 2.12(s, 3H) | 121-.125 |
| 26 | 423.44 | 2.93 | 422 | 1H NMR, 300 MHz, DMSO-d6 (ppm) : | 11.83, 10.96(s, 1H), 8.40-7.06(m, 11H), 5.44, 5.33(s, 2H), 2.40, 2.33(s, 3H), 2.23, 2.12(s, 3H) | 165-166 |
| 27 | 361.47 | 2.80 | 362 | 1H NMR, 300 MHz, DMSO-d6 (ppm) : | 11.30, 10.83(s, 1H), 8.50-7.05(m, 10H), 5.43, 5.30(s, 2H), 2.44, 2.30(s, 3H), 2.22, 1.98(s, 3H) | 170-172 |
| 28 | 416.44 | 2.83 | 415 | 1H NMR, 300 MHz, DMSO-d6 (ppm) : | 11.70(bs, 1H), 10.9(bs, 1H), 8.27-7.05(m, 11H), 5.36(s, 2H), 2.34(s, 3H), 2.14(s, 3H) | 202-205 |
| 29 | 399.50 | 2.86 | 398 | 1H NMR, 300 MHz, DMSO-d6 (ppm) : | 11.43, 10.46(s, 1H), 8.7(bs, 1H), 8.17-7.05(m, 9H), 5.42, 5.29(s, 2H), 2.43, 2.30(s, 3H), 2.21 (s, 6H), 2.19, 2.09(s, 3H) | 237-240 |
| 30 | 567.70 | 3.04 | 566 | 1H NMR, 300 MHz, DMSO-d6 (ppm) : | 11.53, 10.67(s, 1H), 8.24-7.05(m, 20H), 5.43, 5.31(s, 2H), 5.18(s, 4H), 2.38, 2.31 (s, 3H), 2.21, 2.09(s, 3H) | 159-160 |
| 31 | 398.52 | 2.87 | 397 | 1H NMR, 300 MHz, DMSO-d6 (ppm) : | 11.43, 10.43(s, 1H), 8.20-6.72(m, 11H), 5.42, 5.29(s, 2H), 2.96(s, 6H), 2.43, 2.29(s, 3H), 2.21, 2.08(s, 3H) | 219-221 |
| 32 | 400.44 | 2.84 | 399 | 1H NMR, 300 MHz, DMSO-d6 (ppm) : | 11.88, 10.99(s, 1H), 8, 75-7.06(m,103-109.5 11H), 5.45, 5.33(s, 2H), 2.43, 2.33(s, 3H), 2.24, 2.12(s, 3H) | |
| 33 | 401.47 | 2.76 | 400 | 1H NMR, 300 MHz, (ppm): | 11.47, 10.61 (s, 1H), 9.38(bs, 1H), DMSO-d68.23-6.79(m, 10), 5.42, 530(s, 2H), 3.86, 3.81(s, 3H), 2.47, 2.30(s, 3H), 2.21, 2.09(s, 3H) | 220-221 |
| 34 | 397.53 | 2.93 | 398 | 1H NMR, 300 MHz, DMSO-d6 (ppm) : | 11.60, 10.69(s, 1H), 8.30-7.05(m, 11H), 5.43, 5.31 (s, 2H), 2.91 (m, 1H), 2.42, 2.31 (s, 3H), 2.22, 2.10(s, 3H), 1.22(d, 6H) | 196-197 |
| 35 | 380.46 | 2.83 | 381 | 1H NMR, 300 MHz, DMSO-d611H), (ppm) : | 11.87, 11.0(s, 1H), 8.35-7.06(m, 5.36(s, 2H), 2.35(s, 3H), 2.15(s, 3H) | 223.5-225 |
| 36 | 419.92 | 2.86 | 420 | 1H NMR, 300 MHz, DMSO-d6 (ppm) : | 11.50, 10.52(s, 1H), 8.62(bs, 1H), 8.18-7.26(m, 8H), 2.54, 2.37(s, 3H), 2.20(s, 6H), 2.20, 2.09(s. 3H) | 139-212 |
| 37 | 400.87 | 2.92 | 399. | 1H NMR, 300 MHz, DMSO-d6 (ppm) : | 12(ds, 1H), 8.38-7.39(m, 10H), 2.41 (s, 3H), 2.14(s, 3H) | 226-227.5 |
| 38 | 495.77 | 3.45 (46.65 %), 3.75 (45.64 %) | 493 | 1H NMR, 300 MHz, DMSO-d6 (ppm) : | 12.45(s, 1H), 10.58(s, 1H), 8.69(s, 1H), 7.98(s, 1H), 7.60(m, 3H), 7.42(m, 2H), 6.83(d, 1H), 2.57(s, 3H), 2.19(s, 3H) | 292-294.5 |
| 39 | 416.87 | 3.20 (70.73 %), 3.43 (29.27 %) | 415 | 1H NMR, 300 MHz, DMSO-d6 (ppm) : | 12.31(s, 1H), 10.46(s, 1H), 7.94(s, 1H), 7.60(m, 3H), 7.46(s, 1H), 7.25(t, 1H), 7.00(t, 1H), 6.87(d, 1H), 2.50(s, 3H), 2.18(s, 3H) | 8.44(d,281-293 |
| 40 | 451.32 | 3.42 (36.77 %), 3.69 (53.69) | 449 | 1H NMR, 300 MHz, DMSO-d6 (ppm): | 12.3(bs, 1H), 10.58(s, 1H), 7.98(s, 1H), 7.60(m, 3H), 745(s, 1H), 7.28(d, 1H), 6.87(d, 1H), 2.58(s, 3H), 219(s, 3H) | 8.53(s, 914-315 |
| 41 | 485.76 | 3.52 | 483 | 1H NMR, 300 MHz, DMSO-d6 (ppm) : | 13.89(s, 1H), 11.81 (bs, 1H), 8.41(s, 1H), 7.61(m, 3H), 7.43(m, 2H), 7.14(d, 1H), 2.58(s, 3H), 2.24(s, 3H) | 284-285 |
| 42 | 430.90 | 3.28 (68.85 %), 3.55 (31.15 %) | 431 | 1H NMR, 300 MHz, DMSO-d6 (ppm) : | 12.32(bs, 1H), 10.35(s, 1H), 8.37(s, 1H), 7.93(s, 1H), 7.60(m, 3H), 7.44(dd, 1H). 7.06(d, 1H). 6.75(d, 1H), 2.59(s, 3H), 2.28(s, 3H), 2.18(s, 3H) | 302-303 |
| 43 | 434.86 | 3.29 (67.04 %). 3.51 (31.34 %) | 435 | 1H NMR, 300 MHz, DMSO-d6 (ppm) : | 12.2(bs, 1H), 10.65(s, 1H), 8.44(s, 1H), 8.00(s, 1H), 7.60(m, 3H), 7.46(s, 1H), 7.26(d, 1H), 6.94(d, 1H), 2.54(s, 3H), 2.19(s, 3H) | 271-279 |
| 44 | 500.87 | 3.45 (71.15 %), 3.67 (28.85 %) | 499 | 1H NMR, 300 MHz, DMSO-d6 (ppm) : | 12.2(bs, 1H), 10.65(s, 1H), 8.44(s, 1H), 8.00(s, 1H), 7.60(m, 3H), 7.46(s, 1H), 7.26(d, 1H), 6.94(d, 1H), 2.54(s, 3H), 2.19(s, 3H) | 262-276 |
| 45 | 475.35 | 3.27 (28.50 %), 3.64 (66.65 %) | 473 | 1H NMR, 300 MHz, DMSO-d6 (ppm) : | 12.3(bs, 1H), 10.56(s, 1H), 8.67(s, 1H), 8.06(s, 1H). 7.32(m, 4H), 7.09(d, 2H), 6.82(d, 1H), 5.42(s, 2H), 2.50(s, 3H), 2.21 (s, 3H) | 288-290 |
| 46 | 396.46 | 3.00 (27.06 %), 3.35 (71.55 %) | 397 | 1H NMR, 300 MHz, DMSO-d6 (ppm) : | 12.26(bs, 1H), 10.43(s, 1H), 8.42(s, 1H), 8.02(s, 1H), 735-6.84(m, 8H), 5.41(s, 2H), 2.50(s, 3H), 2.20(s, 3H) | 292-293 |
| 47 | 476.52 | 3.19 (26.81 %), 5.68 (65.39 %) | 475 | 1H NMR, 300 MHz, DMSO-d6 (ppm) : | 12.23(bs,1H), 10.44(s, 1H), 8.81 (s, 1H), 8.00(s, 1H). 7.49(dd, 1H), 7.29(m, 3H), 7.09(d, 2H), 6.76(d, 1H), 5.42(s, 2H), 2.49(s, 3H), 2.20(s, 3H) | bomlik |
| 48 | 430.90 | 3.24 (27.04 %), 3.59 (66.89 %) | 429 | 1HNMR.300 MHz, DMSO-d6 (ppm) : | 12.3(bs, 1H), 10.55(bs, 1H), 8.51 (s, 1H). 8.06(s, 1H), 7.28(m, 4H), 7.07(m, 2H), 6.86(d, 1H). 5.42(s, 2H), 4.99(s, 3H), 2..49(s, 3H), 2.21(s, 3H) | 302-303 |
| 49 | 465.35 | 3.38 | 465 | 1HNMR.300 MHz, DMSO-d6 (ppm) : | 13.79(s, 1H), 12(bs,1H), 8.46(s, 1H), 7.26(m, 4H), 7.08(d, 2H)., 6.96(s, 1H), 5.46(s, 2H), 2.50(s, 3H), 2.26(s, 3H) | 299 |
| 50 | 465.35 | 3.34 | 465 | 1H NMR,300 MHz, DMSO-d61H), (ppm) : | 13.86(s, 1H), 11.82(s, 1H). 8.48(s, 298 7,40-7.07(m, 7H), 5.46(s, 2H), 2.49(s, 3H), 2.26(s, 3H) | |
| 51 | 450.59 | 3.49 | 449 | 1HNMR.300 MHz, DMSO-d62H), (ppm) : | 8.23(s, 1H), 8.05(s, 1H), 7.62(d, 7.40-7.22(m, 6H), 7.03(d, 2H), 6.44(s, 1H). 5.36(s, 2H), 2.26(s, 3H), 2.16(s, 3H), 1.33(s, 9H) | 165-168 |
| 52 | 495.59 | 3.57 | 494 | 1H NMR, 300 MHz, DMSO-d6 2H), (ppm) : | 8.4(bs, 1H), 8.24(d, 2H), 8.02(d. 7.99(s, 1H). 7.25(m, 3H), 7.04(d, 2H), 6.50(s, 1H), 5.36(s, 2H), 2.36(s, 3H), 2.18(s, 3H), 1.33(s, 9H) | 219.5-221 |
| 53 | 495.59 | 3.53 | 494 | 1H NMR, 300 MHz, DMSO-d6 (ppm) : | 8.51 (s, 1H). 8.05(d, 1H), 7.98(s, 1H), 7.65(t, 1H), 7.26(m, 3H), 7.00(m, 2H), 6.46(s, 1H), 5.36(s, 2H), 2.34(s, 3H), 2.16(s, 3H), 1.34(s, 9H) | >200 decomposition |
| 54 | 468.5 | 3.54 | 467 | 1H NMR, 300 MHz, DMSO-d6 (ppm) : | 8.34(s, 1H), 8.35(s, 1H), 8.47-7.20(m, 6H), 7.05(m. 3H), 6.44(s, 1H), 5.37(s, 2H), 2.36(s, 3H), 2.17(s, 3H), 1.32(s, 9H) | 153-156 |
| 55 | 471.0 | 3.57 | 471 | 1H NMR, 300 MHz, DMSO-d6 (ppm) : | 8.33(s, 1H), 8.00(s, 1H), 7.65-7.52(m, 4H), 7.37(m, 6H), 7.25(t, 1H), 6.44(s, 1H), 2.34(s, 3H), 2.15(s, 3H) | 169-176 |
| 56 | 489.0 | 3.62 | 489 | 1H NMR, 300 MHz, DMSO-d6 (ppm) : | 8.43(s, 1H), 7.98(s, 1H), 7.60-7.36(m, 7H), 7.09(t, 1H), 6.45(s, 1H), 2.38(s, 3H), 2.17(s, 3H), 1.33(s, 9H) | 189-191.5 |
| 57 | 516.0 | 3.64 | 514.2 1H | NMR, 300 MHz, DMSO-d6 (ppm) : | 8.60(s, 1H), 8.27(d, 2H), 7.95(m, 3H), 7.56(m, 3H), 7.38(m, 1H), 3.50(s, 1H). 2.42(s, 3H), 2.18(s, 3H), 1.34(s, 9H) | 195-198 |
| 58 | 494.6 | 3.51 | 495.2 1H 1 | NMR, 300 MHz, DMSO-d6 (ppm) : | 8.32(s, 1H), 8.07(s, 1H), 7.42(m, 3H), 7.26(m, 3H), 7.07(m, 3H), 6.43(s, 1H), 5.31 (s, 2H), 2.69(bs, 2H), 2.49(bs, 2H), 1.71 (bs, 4H), 1.32(s,9H) | 147-153 |
| 59 | 476.6 | 3.47 | 475.2 1H 6 | NMR, 300 MHz, DMSO-d6 (ppm) : | 8.21(s, 1H), 8.09(s, 1H), 7.59(d, 2H), 7.41-7.20(m, 6H), 7.08(d, 2H), 6.41 (s, 1H), 5.30(s, 2H), 2.63(bs, 2H), 2.49(bs, 2H), 1.70(bs, 4H), 1.32(s, 9H) | 188-190 |
| 60 | 501.4 | 2.92 | | 1H NMR, CDCl3 ppm | 10.10; 9.80 (s, 1H, NH), 7.51-7.19 (m, 9H, aromatic-H), 2.55 (s, 3H, CH3), 2.36 (s, 3H, CH3), 2.30 (s, 3H, CH3), 2.24 (s, 3H. CH3) | 234,3-237,2 |
| 61 | 507.0 | 2,98 | 507 | 1H NMR, CDCl3 ppm | 10.06; 9.84 (s, 1H, NH), 7.50-7.22 (m, 10H, aromatic-H), 5.38 (s, 2H, CH2), 3.09; 2.90 (bs, 2H, 1"'- H2), 2.61; 2.59 (s, 3H, CH3), 2.57 (bs, 2H, 4"'- H2), 2.27; 2.24 (s, 3H, CH3),1.88; 1.80 (bs, 4H, 2"'-, 3"'- H2) | 203,7-204,7 |
| 62 | 605.1 | 3,06 | 605 | 1H NMR, CDCl3 ppm | 10.10 (bs, 1H, NH), 7.53-7.04 (m, 10H, aromatic-H), 5.43 (s, 2H, CH2),2.60 (s, 2H, CH2),2.27 (s, 3H, CH3) | 187,4-189,3 |
| 63 | 481.0 | 2,94 | 481 | 1H NMR, CDCl3 ppm | 10.10 (s, 1H, NH), 7.50-7.18 (m, 10H, aromatic-H), 5.46 (s, 2H, CH2), 2.60 (s, 3H, CH3), 2.46 (s, 3H, CH3), 2.28 (s, 3H, CH3), 2.26 (s, 3H, CH3) | 228,1-229,0 |
| 64 | 503.0 | 3,06 | 502 | 1H NMR, CDCl3 ppm | 10.45 (s, 1H. NH), 8.20 (d, 1H, 7-H, 3J=7.8 Hz), 7.61-7:25 (m, 13H, aromatic-H), 5.72 (s, 2H, CH2), 2.56 (s, 3H, CH3), 2.28 (s, 3H, CH3) | 252,2-253,1 |
| 65 | 508.6 | 2,97 | 509 | 1H NMR, CDCl3 ppm | 10.64 (s, 1H, NH), 8.55 (d, 1H, 7-H, 3J=7.8 Hz), 7.55-7.21 (m, 14H, aromatic-H), 5.75 (s, 2H, CH2), 5.50 (s, 2H, CH2), 4.93 (bs, 2H, 1'- H2), 2.61 (bs, 2H, 4'-H2), 1.91 (bs, 2H, 2'-,3'- H2), | 254,6-255,6 |
| 66 | 606.7 | 3,03 | 607 | 1H NMR, CDCl3 ppm | 10.43 (s, 1H, NH), 8.56 (d, 1H, 7-H, 3J=7.8 Hz), 7.50-7.13 (m, 24H, aromatic-H), 5.76 (s, 2H, CH2), 5.53 (s, 2H, CH2) | decomposition >90 |
| 67 | 482.5 | 2,89 | 483 | 1H NMR, CDCl3 ppm | 10.47; 10.20 (s, 1H, NH), 8.54; 8.42 (d, 1H. 7-H, 3J=7.8 Hz), 7.60-7.16 (m, 14H, aromatic-H), 5.73 (s, 2H, CH2), 5.69; 5.53 (s, 2H, CH2), 2.46 (s, 3H, CH3), 2.23 (s, 3H, CH3) | decomposition >90 |
| 68 | 507.0 | 3,01 | 507 | 1H NMR, CDCl3 ppm | 10.05; 9.87 (s, 1H, NH), 7.54-7.00 (m, 10H, aromatic-H), 5.41; 5.40 (s, 2H, CH2), 2.86 (bs, 2H, 7- H2), 2.63 (bs, 2H, 10- H2), 2.50 (s, 3H, CH3), 2.49 (s, 3H, CH3), 1.78 (bs, 4H, 8-,9- H2) | 220,1-221,5 |
| 69 | 512.6 | 3,02 | 513 | 1H NMR, CDCl3 ppm | 10.10; 9.92 (s, 1H, NH), 7.33-7.11 (m, 11H, aromatic-H), 5.41 (s, 2H, CH2), 5.33 (s, 2H, CH2), 3.06; 2.90 (bs, 4H, 1'-,1"-H2), 2.57 (bs, 4H, 4'-,4"- H2), 1.86 (bs, 8H, 2'-,2"-,3'-,3"- H2) | 216,219,9 |
| 70 | 610.7 3,08 | | 611 | 1H NMR, CDCl3 ppm | 10.2 (bs,1H,NH), 7.33-7.03 (m, 21H, aromatic-H), 5.46 (s, 2H, CH2), 5.40 (s, 2H, CH2), 2.93 (bs, 2H, 7- H2), 2.58 (bs, 2H, 10- H2),1.77 (bs, 4H, 8-,9- H2) | 191,0-191,7 |
| 71 | 486.6 | 2,99 | 487 | 1H NMR, DMSO d6 ppm | 9.67 (s, 1H, NH), 7.35-7.07 (m, 11H, aromatic-H), 5.42 (s, 2H, CH2), 5.32 (s, 2H, CH2), 2.92 (bs, 2H, 7-H2), 2.50 (bs, 5H, 10- H2, CH3), 2.49 (s,3H, CH3), 1.74 (bs, 4H, 8-, 9- H2) | 225-229,5 |
| 72 | 481.0 | 2,88 | 481 | 1H NMR, CDCl3 ppm | 9.98 (s, 1H, NH), 7.51-7.03 (m, 10H, aromatic-H), 5.46 (s, 2H, CH2), 2.51 (s, 3H, CH3), 2.29 (s, 3H, CH3), 2.24 (s, 3H, CH3), 2.19 (s, 3H, - CH3) | 237.9-240,5 |
| 73 | 486.6 | 3,06 | 487 | 1H NMR, CDCl3 ppm | 10.00; 9.90 (s,1H, NH), 7.32-7.06 (m,11H, aromatic-H), 5.48; 5.45(s, 2H, CH2), 5.35; 5.32(s,2H, CH2), 3.12; 2.90 (bs,2H, 1"- H2), 2.55 (bs, 2H, 4"- H2),2.52(s, 3H, CH3),2.24(s, 3H, CH3), 1.86 (bs, 4H, 2"-,3"-H2) | 248,9-249,6 |
| 74 | 584.7 | 3,11 | 585.2 9 | 1H NMR, CDCl3 ppm | 10.01 (bs, 1H, NH), 7.31-7.04 (m, 21H, aromatic-H), 5.50 (s,2H, CH2), 5.41 (s, 2H, CH2), 2.53 (s, 3H, CH3) , 2.26 (s, 3H, CH3) | 197,1-197,6 |
| 75 | 460.5 | 2,88 | 461 | 1H NMR, CDCl3 ppm | 10.11; 9.90 (s, 1H, NH), 7.33-7.09 (m, 11H, aromatic- H),5.50; 5.46 (s, 2H, CH2), 5.40 (s, 2H, CH2), 2.51 (s, 3H, CH3),2.44 (s, 3H; CH3), 2.25 (s, 3H, CH3),2.22 (s, 3H, CH3) | 236,0-239,1 |
| 76 | 248.3306 | 1.56 | 249 | 1H NMR, 300 MHz, DMSO-d6 (ppm): | 11.63(s, 1H), 7.74(s, 1H),4.05(t, 2H), 2.22(s, 3H), 2.2 1(s, 3H), 2.18(t, 2H), 2.11(s, 6H), 1.74(q,2H) | 165-166.5 |
| 77 | 77266.7762 | 2.22 | 267 | 1H NMR, 300 MHz,DMSO-d6 (ppm): | 8.47(s, 1H), 4.23(t, 2H), 2.40(s, 3H), 2.36(s, 3H), 2.21 (t, 2 H), oil 2.13(s, 6H), 1.80(q,2H) | |
| 78 | 262. 3605 | 0.56 | 263 | 1H NMR, 300 MHz, DMSO-d6 (ppm): | 8.86(bs, 1H), 8.12, 7.67(s,1H),4.39, 4.11 (bs, 2H), 4.07(t, 2H), 2.27(s, 3H), 2.25(s, 3H), 2.17(t, 2H), 2.10(s, 6H), 1.77-1.71(q, 2H) | 90-92 |
| 79 | 396.4963 | 2.02 | 397 | 1H NMR, 300 MHz, DMSO-d6 (ppm): | 11.36, -10.47(s, 1H), 9.27(bs, 1H), 8.19-6.76( m, 5H), 4.13, 4.01(t, 2H), 3.84, 3.79(s, 3H), 2.48-2.11(m, 14H), 1.76(q, 2H) | 195.6-196.4 |
| 80 | 382.4692 | 1.91 | 383 | 1H NMR, 300 MHz, DMSO-d6 (ppm): | 11.30, 10.25(bs, 1H), 9.11(bs, 2H), 8.12-6.73(m, 5H), 4.13, 4.01(t, 2H), 2.48-2.11(m, 14), 1.76(q,2H) | 185 |
| 81 | 475.6422 | 2.92 | 476 | 1H NMR, 300 MHz, DMSO-d6 (ppm): | 8.04(s, 1H), 7.99(s,1H), 7.46(d, 2H), 6.92(d, 2H), 6.36(s, 1H), 4.07(t, 2H), 3.71(s, 3H), 2.26(s, 3H), 2.21(s, 3H), 2.16(t, 2H), 2.09(s, 6H), 1.72(q, 2H), 1.30(s, 9H) | 91.7-93.6 |
| 82 | 475.6422 | 2.99 | 476 | 1H NMR, 300 MHz, DMSO-d6 (ppm) : | 8.12(s, 1H), 8.03(s, 1H), 7.26(t, 1H), 7.16(m, 2H), 6.80(d, 1H), 6.39(s, 1H), 4.08(t, 2H), 3.60(s, 3H), 2.27(s, 3H), 2.24(s, 3H), 2.15(t, 2H), 2.09(s, 6H), 1.72(q, 2H), 1.30(s; 9H) | 102-104 |
| 83 | 490.6133 | 3.04 | 491 | 1H NMR, MHz, DMSO-d6 (ppm): | 300 8.61-7.64(m, 6H), 6.45(s, 1H), 4.08(t, 2H), 2.32(s, 3H), 2.28(s, 3H), 2.16(t, 2H), 2.10(s, 6H), 1.73(q, 2H), 1.33(s, 9H) | 164.4-166 |
| 84 | 226.2833 | 3.30 | 227 | 1H NMR, MHz, DMSO-d6 (ppm): | 300 7.86(t,1H),7.29(d, 1H), 7.17(d, 1H), 5.67(s, 2H), 2.49(s, 3H), 1.92(s, 3H) 1.87(s, 3H) | oil |
| 85 | 254.2939 | 2.59 | 255 | 1H NMR, MHz, DMSO-d6 (ppm): | 300 11.82(s,1H),7.88(t, 1H), 7.71 (s, 1H), 7.33(d, 2H), 2.49(s, 3H), 2.28(s, 3H), 2.14(s, 3H) | 298.5-300 |
| 86 | 272.7395 | 3.79 | 273 | 1H NMR, 300 MHz, DMSO-d6 (ppm): | 8.47(s, 1H), 7.97(t, 1H), 7.49(d, 1H), 7.44(d, 1H), 2.53(s, 3H), 2.45(s, 3H), 2.34(s, 3H) | 198.3-198.7 |
| 87 | 268.3238 | 2.05 | 269 | 1H NMR, 300 MHz, DMSO-d6 (ppm): | 8.10(s,1H),7.89(t, 1H), 7.86(bs, 1H), 7.4 1(d,1H), 7.3 1(d, 1H),4.47(s,2H),2.50(s,3H),2.35(s,3H),2.23(s,3H) | 214.3-215.5 |
| 88 | 388.4326 | 2.60 | 389 | 1H NMR, 300 MHz, DMSO-d6 (ppm): | 11.50, 10.45(s, 1H), 9.1(bs, 2H), 8.18-6.77(m, 8H), 2.54, 2.36(s*,* 3H), 2.28(s, 3H), 2.17(s, 3H) | 264.2-264.8 |
| 89 | 417.4307 | 2.88 | 418 | 1H NMR, 300 MHz, DMSO-d6 (ppm): | 11.74(s, 1H), 11.27, 10.74(bs, 1H),8.38-7.16(m,8H),2.45, 2.27(s, 3H), 2.4(s, 3H), 2.19(s, 3H) | 216.5-217 |
| 90 | 496.5766 | 4.65 | 497 | 1H NMR, 300 MHz, DMSO-d6 d6 (ppm): | 8.6-7.26(m, 9H), 6.51(s,1H), 2.53(s, 3H), 2.40(s, 3H), 2.26(s, 3H), 1.34(s, 9H) | 108-110 |
| 91 | 481.6056 | 4.49 | 482 | 1H NMR, 300 MHz, DMSO-d6 (ppm): | 8.23(s, 1H), 8.04(s, 1H), 7.89(t, 1H), 7.52(d, 2H), 7.41(d, 1H), 7.32(d, 1H), 6.95(d, 2H), 6.41 (s, 1H),3.73(s, 3H), 2.53(s, 3H), 2.3 1(s, 3H), 2.24(s, 3H), 1.32(s, 9H) | 221.5-222.4 |
| 92 | 517 | 4.49 | 482 | 1H NMR, 300 MHz, DMSO-d6 (ppm): | 8.23(s, 1H), 8.04(s,1H), 7.89(t, 1H), 7.52(d, 2H), 7.41(d, 1H), 7.32(d, 1H), 6.95(d, 2H), 6.41(s, 1H), 3.73(s, 3H), 2.53(s, 3H), 2.31(s, 3H), 2.24(s, 3H), 1.32(s, 9H) | 97-98 |

### Example 93. (Study of pharmaceutical effect)

### Examination of active substances inhibiting release of substance P (SP) by radio immuno-assay (RIA) method in vitro on rat trachea

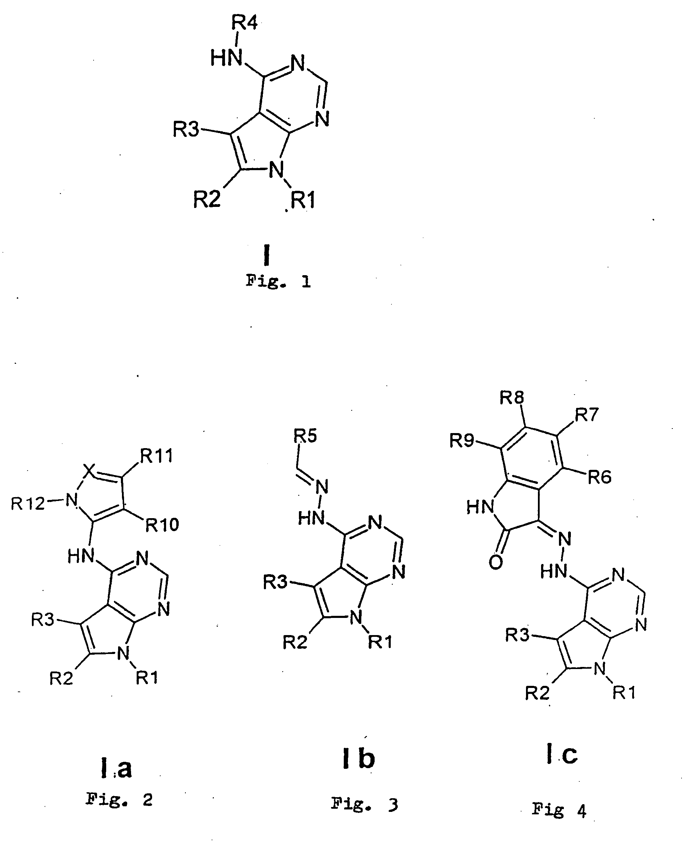

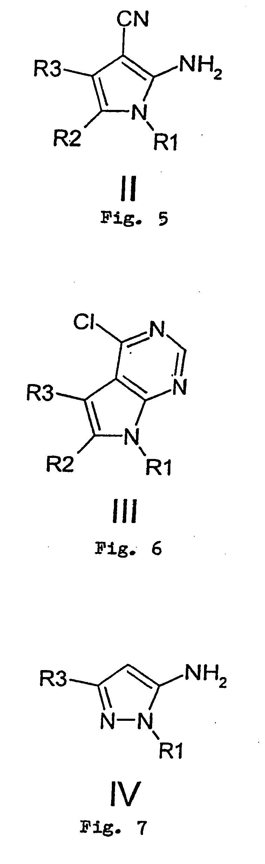

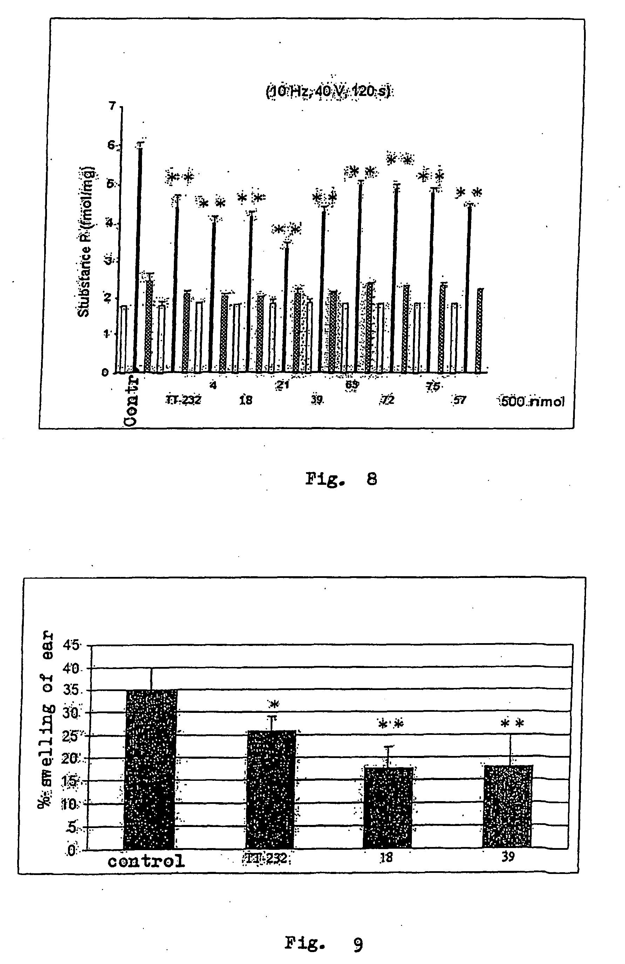

The influence of active substances on the SP release (of 500 nanomoles) was examined in in vitro environment on rat trachea in biological bath (Wistar's rats of 200 to 250 g weight were used). Samples were taken three times in periods of 8 minutes after perfusion with oxygenated Krebs's solution for 1 hour. The organ was excited with electric current of 10 Hz, 40 V, 0.1 ms, 120 s in the middle period of 8 minutes. The SP content of the samples were measured by a specific radio immuno-assay method. The values are represented in the diagram in the following way: the value before electric excitation with empty column, the one measured immediately after excitation with black and the one measured at 8 minutes after excitation with shaded columns. The following radio immuno-assay method was used: 5000 cpm of mono-iodized SP marked with I¹²⁵ iodine isotope was used as RIA tracer. Synthetic SP was used as standard in the concentration range of 0 to 100 phento moles per ml. The neuropeptide concentrations related to the weight of humid tissues of the samples were expressed in units "phento moles per mg". The Figure 8 shows the inhibiting effect of TT-232 as reference substance and 8 compounds in concentration of 500 nM to the release of P-substance under electric excitation (see the black columns). The significant values related to the control ones received on prepared organs treated only with solvent are shown.

### Example 94. (Study of pharmaceutical effect)

### Examination of the in vivo neurogenic inflammation inhibiting effect.

### (Swelling and plasm protein extravasation provoked by capsaicine in the mouse ear).

BALB/c male mice of 18 to 20 g were anesthetized with ketamine (100 mg/kg according to the invention) and xylazine (10 mg/kg i. m.). Having measured the ear thickness at the base the examined substances were applied according to the invention in quantities of 0.1 ml per 10 g. 10 minutes later Evans blue stain of 125 mg per kg (solution of 2.5 %, 0.05 ml per 10 g) was injected into tail veins then 5 minutes later 10 µl of alcohol of 96 % was applied on the left ear and 10 µl of capsaicine solution of 2.5 % was applied on the right ear of each animal. The Evans blue marker stain connects itself to the plasma albumin in the circulation. The surfaces become blue where the plasma albumin leaves blood vessels because of inflammation. The thickness of ears was measured again after 30 minutes and the swelling was defined as per cents of the initial thickness. The animals were killed thereafter by bleeding to death, their ears were cut and the mass of the ears was measured. The stain accumulated in the tissue pieces was extracted with 1.5 ml of formamide at 20 C° for 72 hours. The optical densities of the solutions were determined by spectrophotometry (with microplate reader) at the wavelength of 620 nm. The quantity of extravased stain (indicating the plasma albumin quantity) was defined in units of mg per humid tissue The values measured in the ears treated in alcohol were subtracted from the ones measured in the ears treated with capsaicine at both animals. The animals treated with solvent served as control. The Figure 9 shows accumulation of Evans blue quantity in the control group pretreated with solvent (black column) after administering TT-232 as reference substance and dosing of 100 µg/kg of 11 compounds according to the invention. Non-parametric Mann-Whitney test was used to the statistical evaluation of the results. *p < 0.05; **p < 0.01.

### Example 95. (Study of pharmaceutical effect)

### Examination of vasodilatation and extravasation of plasma protein in mouse's ear provoked by mustard oil.

Balb/c male mice were anesthetized with ketamine (100 mg/kg according to the invention) and xylazine (5 mg/kg i. m.) then albumin (30 kBq activity per mouse) marked with 0.1 ml of iodine isotope I¹²⁵ was dosed i. v. 10 µl of mustard oil of 1 % was dribbled onto both sides of the left ear of each mouse and smeared. The radioactivity was measured in each minute for 50 minutes with gamma ray counter above the ear. The increase of activity indicates vasodilatation and extravasation of the plasma protein. 20 µg/kg of TT-232 and non peptide following molecule 11527 (10 and 100 µg/kg) were dosed according to the invention 30 minutes before the treatment with mustard oil. A solvent was used in the control group.

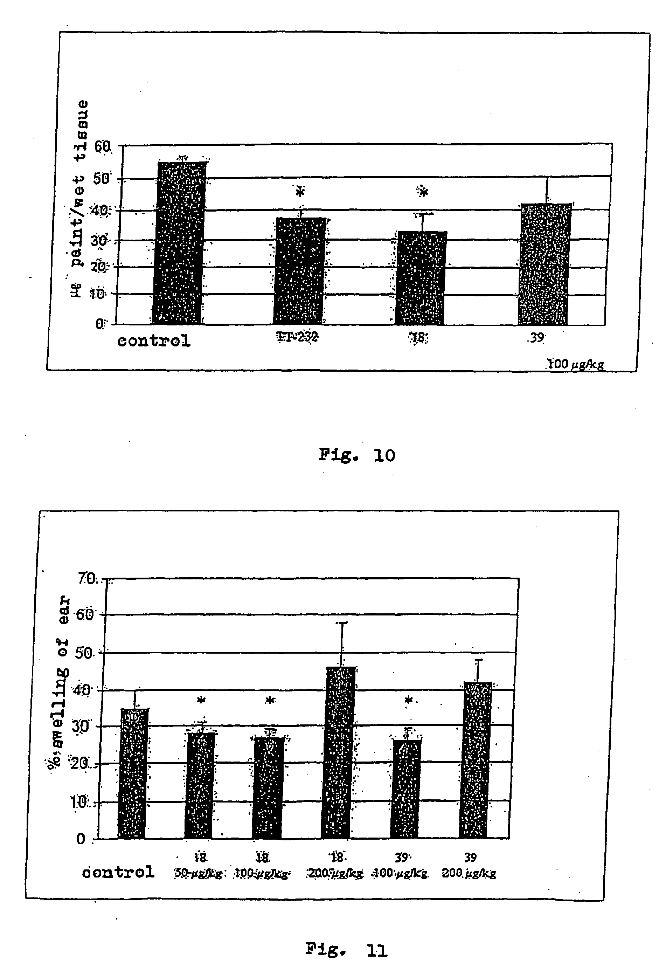

Bonferroni's modified test after ANOVA was used for statistical evaluation, *p < 0.05, **p < 0.01; n=7 per group (see Figure 10).

### Example 96. (Study of pharmaceutical effect)

### Examination of neuropathic hyperalgesia

The partial unilateral ligation of n. ishiadicus causes diminution of the mechanonociceptive threshold of the extremity (Seltzer's operation). The mechanonociception was examined with Ugo Basile's analgesimeter (Randall-Selitto test). The foot of the animal was put under a teflon cone with rounded end and gradually increasing force was exerted onto middle region of the beck of foot. The pain threshold is the value when the animal pulls its leg out. It may be read out in grams by the hand moving before linear scale. The change of the mechanical threshold was expressed in per cents of the own initial threshold. The nervus ischiadus of the thigh of male Sprague-Dawley rats was prepared on the one side in pentobarbital narcosis after control measurements then 1/3 to 1/2 part of the nerves were carefully detached, tightly bound with non-traumatic thread (Mersilene, Ethicon) of 6/0 size, then the incision was closed. The mechanonociceptive thresholds were measured again on the 7^{th} day after the operation. Only the animals were involved in the following examination, which showed reduction of thresholds of at least 20% related to the control results. The measurements were repeated 20 minutes after dosing of 18 according to invention. The results were expressed in per cents related to the initial values measured before dosing (shown by white column) and compared to the control group with solvent. Non-parametric Mann-Whitney tests were used to determine statistically significant differences. *p < 0.05, **p < 0.1, n = 6 to 8 per group (see Figure 11).

## Claims

1. 7H-pyrrolo[2,3-d]pyrimidine derivatives of the general formula (I), as well as their pharmaceutically acceptable salts **characterized by** that in the formula 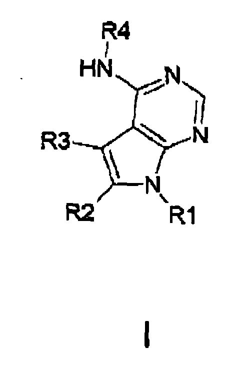
R1 is alkyl, aryl, heteroaryl, aryl-alkyl with 1-4 carbon atoms, heteroaryl- alkyl with 1-4 carbon atoms, morpholino-alkyl with 1-4 carbon atoms or dialkylamino-alkyl withl-4 carbon atoms,
R2, R3 independently of each other are hydrogen, methyl, ethyl, propyl, isopropyl or cyclopropyl groups or R2 and R3 are together a tetramethylene group
R4 is 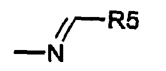 or 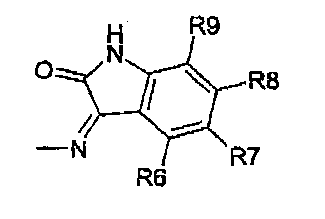 or 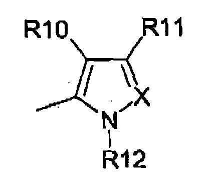 group, wherein
R5 is a substituted or unsubstituted aromatic or heteroaromatic ring where
R6, R7, R8 and R9 independently of each other are hydrogen, halogen, nitro, amino, alkylamino, dialkylamino, hydroxy, methoxy, ethoxy, isopropoxy or sulfonyl group,
R10 is hydrogen or nitrile group,
R11 is hydrogen, methyl, ethyl, propyl, isopropyl, tert.-butyl group or tetramethylene ring connected to X,
R12 is alkyl, aryl, heteroaryl, aryl-alkyl with 1-4 carbon atoms, morpholino- alkyl with 1-4 carbon atoms, dialkylamino-alkyl with 1-4 carbon atoms,
X is carbon, if R11 is a tetramethylene ring connected to X, otherwise nitrogen, methane, methyl-methine, ethyl-methine, propyl-methine, isopropyl-methine, cyclopropyl-methine, tert-butyl-methine or phenyl- methine.

2. The compounds of general formula (I) according to Claim 1 **characterized by** that their structures correspond to the general formula (Ia) 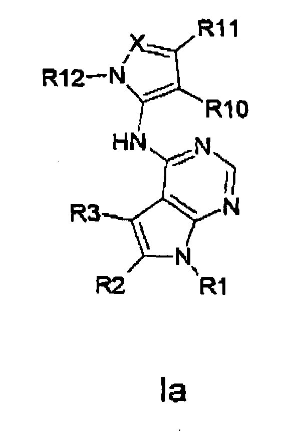 wherein R1, R2, R3, R10, R11, R12 and X are the same as in the Claim 1.

3. The compounds of general formula (I) according to Claim 1 **characterized by** that their structures correspond to the general formula (Ib) 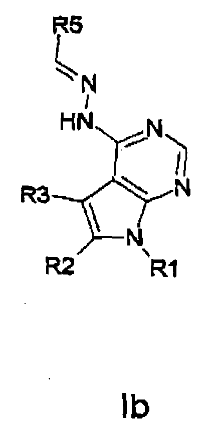 wherein R1, R2, R3 and R5 are the same as in the Claim 1.

4. The compounds of general formula (I) according to Claim 1 **characterized by** that their structures correspond to the general formula (Ic) 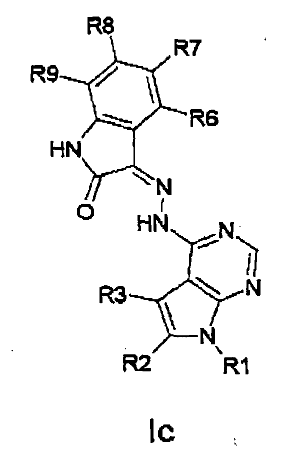 wherein R1,R2, R3, R6, R7, R8 and R9 are the same as in the Claim 1.

5. Pharmaceutical preparations **characterized by** that they contain compounds of general formula (I) and therapeutically acceptable additives.

6. Pharmaceutical preparations according to Claim 5 **characterized by** that they might be applied as antiphlogistic or analgetic medicament.

7. Pharmaceutical preparations according to Claim 5 **characterized by** that they might be applied as neuropathic hyperalgesia reducing medicament.

8. Pharmaceutical preparations according to Claim 5 **characterized by** that they might be applied as rheumatic arthritis reducing medicaments.

9. Pharmaceutical preparations according to Claim 5 **characterized by** that they might be applied as medicament hindering destruction of bones or chondrus.

10. Pharmaceutical preparations according to Claim 5 **characterized by** that they might be applied for treatment of diseases, which may be connected with inflammatory processes e.g. asthma, eczema or psoriasis.

11. Process for producing 7H-pyrrolo[2,3-d]pyrimidine derivatives of the general formula (I) **characterized by** that a compound of general formula (II) 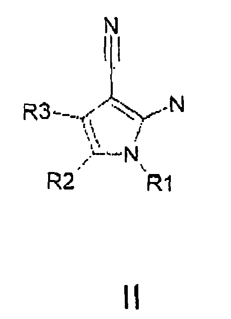 produced from acetoin with amine and malonic acid dinitrile of molar equivalent quantities where R1, R2 and R3 are the same as mentioned in the formula (I),the compound is mixed with formic acid of mass excess of 5 to 10 times at reflux temperature for a time period of 1 hour to 2 days, then the mixture is poured into ice-water, the precipitated product is separated, the product is dried then brought into reaction with phosphorus oxychloride of mass excess of 5 to 10 times at reflux temperature for 0,5 to 4 hours then the mixture is poured onto ice and the precipitated imidoyl-chloride of general formula (III) 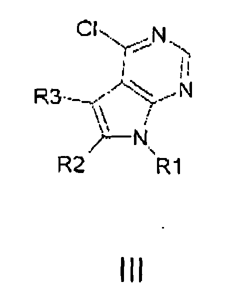 where R1, R2 and R3 are the same as mentioned in the formula (I) is separated, dried and evaporated, thereafter
A) the imidoyl chloride of general formula (III) is solved in an aprotic solvent and brought into reaction with amine of general formula (II) or (IV) 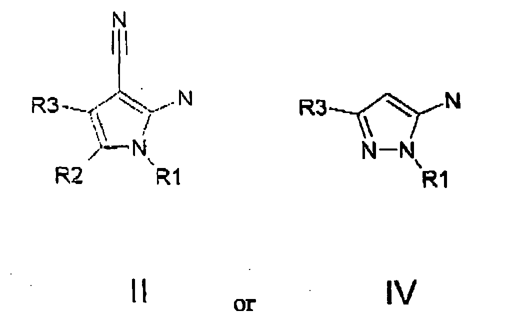 of equivalent quantity where R1 and R3 are the same as above adding NaH of 2 to 10 times of molar equivalent excess for 0.5 to 6 hours, the produced mixture is poured onto ice and the precipitated product is separated and purified,
or
B) the imidoyl chloride of general formula (III) produced in this way is brought into reaction with hydrazine hydrate of molar equivalent excess of 2 to 10 times in a medium of polar organic solvent, the reaction product in the organic phase is separated from the mixture, the organic phase is dried and evaporated then bruised with an apolar solvent, the hydrazine derivative produced in this way is mixed with a polar organic solvent and brought into reaction with an aldehyd of equivalent quantity at 20 to 120 C° for 1 to 12 hours, then the reaction product is separated,
or
C) the hydrazine derivative produced according to version B) is mixed with a polar organic solvent and brought into reaction with isatin of equivalent quantity then the product of reaction is separated.

## Patentansprüche

1. 7H-Pyrrolo[2,3-d]-pyrimidin-Derivate der allgemeinen Formel (I) sowie deren pharmazeutisch verträgliche Salze, **dadurch gekennzeichnet, dass** in der Formel 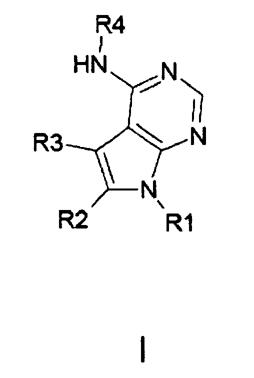
R1 für Alkyl, Aryl, Heteroaryl, Arylalkyl mit 1-4 Kohlenstoffatomen, Heteroarylalkyl mit 1-4 Kohlenstoffatomen, Morpholinoalkyl mit 1-4 Kohlenstoffatomen oder Dialkylamino-alkyl mit 1-4 Kohlenstoffatomen steht,
R2,R3 unabhängig voneinander für Wasserstoff, Methyl-, Ethyl-, Propyl-, Isopropyl- oder Zyklopropylgruppe oder R2 und R3 zusammen für Tetramethylengruppe stehen,
R4 für eine Gruppe der Formel 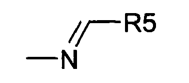 oder 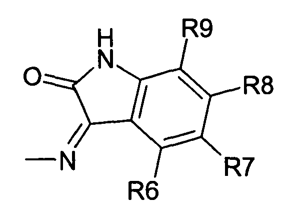 oder 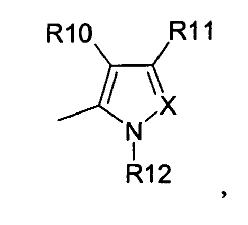 worin
R5 für einen substituierten oder unsubstituierten aromatischen oder heteroaromatischen Ring steht, in dem
R6, R7, R8 und R9 unabhängig voneinander für Wasserstoff, Halogen, Nitro-, Amino-, Alkylamino-, Dialkylamino-, Hydroxy-, Methoxy-, Ethoxy-, Isopropoxy- oder Sulfonylgruppe stehen,
R10 für Wasserstoff oder Nitrilgruppe steht,
R11 für Wasserstoff, Methyl-, Ethyl-, Propyl-, Isopropyl, tert-Butylgruppe oder einen an X gebundenen Tetramethylenring steht,
R12 für Alkyl, Aryl, Heteroaryl, Arylalkyl mit 1-4 Kohlenstoffatomen, Morpholinoalkyl mit 1-4 Kohlenstoffatomen, Dialkylaminoalkyl mit 1-4 Kohlenstoffatomen steht,
X für Kohlenstoff steht, wenn R11 ein an X gebundener Tetramethylenring ist, im anderen Fall für Stickstoff, Methin, Methylmethin, Ethylmethin, Propylmethin, Isopropylmethin, Zyklopropylmethin, tert-Butylmethin oder Phenylmethin steht,

2. Verbindungen der allgemeinen Formel (I) gemäß Anspruch 1, **dadurch gekennzeichnet, dass** ihre Struktur mit der allgemeinen Formel (Ia) 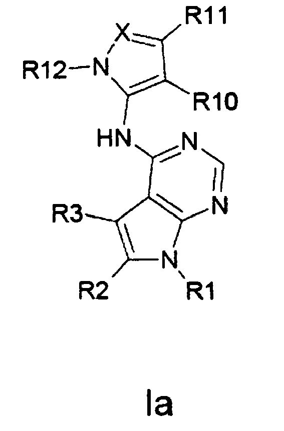 übereinstimmt, worin
R1, R2, R3, R10, R11, R12 und X die im Anspruch 1 angegebene Bedeutung haben.

3. Verbindungen der allgemeinen Formel (I) gemäß Anspruch 1, **dadurch gekennzeichnet, dass** ihre Struktur mit der allgemeinen Formel (Ib) 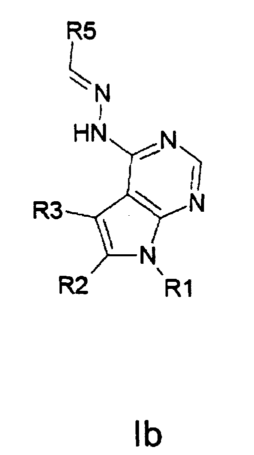 übereinstimmt, worin
R1, R2, R3 und R5 die im Anspruch 1 angegebene Bedeutung haben.

4. Verbindungen der allgemeinen Formel (I) gemäß Anspruch 1, **dadurch gekennzeichnet, dass** ihre Struktur mit der allgemeinen Formel (Ic) 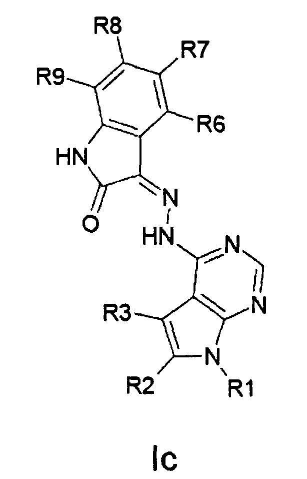 übereinstimmt, worin
R1, R2, R3, R6, R7, R8 und R9 die im Anspruch 1 angegebene Bedeutung haben.

5. Pharmazeutische Zubereitungen, **dadurch gekennzeichnet, dass** sie Verbindungen der allgemeinen Formel (I) und therapeutisch akzeptierbare Zusatzstoffe enthält.

6. Pharmazeutische Zubereitungen gemäß Anspruch 5, **dadurch gekennzeichnet, dass** sie als Antiphlogistikum und Analgetikum angewendet werden können.

7. Pharmazeutische Zubereitungen gemäß Anspruch 5, **dadurch gekennzeichnet, dass** sie als Mittel zur Senkung der neuropathischen Hyperalgesie angewendet werden können.

8. Pharmazeutische Zubereitungen gemäß Anspruch 5, **dadurch gekennzeichnet, dass** sie als Mittel zur Senkung von rheumatischer Arthritis angewendet werden können.

9. Pharmazeutische Zubereitungen gemäß Anspruch 5, **dadurch gekennzeichnet, dass** sie als Mittel zur Hemmung von Knochen- und Knorpelabbau angewendet werden können.

10. Pharmazeutische Zubereitungen gemäß Anspruch 5, **dadurch gekennzeichnet, dass** sie zur Behandlung von Erkrankungen angewendet werden können, die im Zusammenhang mit entzündlichen Prozessen stehen können, z.B. Asthma, Ekzem oder Psoriasis.

11. Verfahren zur Herstellung von 7H-Pyrrolo[2,3-d]-pyrimidin-Derivaten der allgemeinen Formel (I), **dadurch gekennzeichnet, dass** eine aus Acetoin in molaräquivalenter Menge mit Amin und Malonsäure-dinitril hergestellte Verbindung der allgemeinen Formel (II) 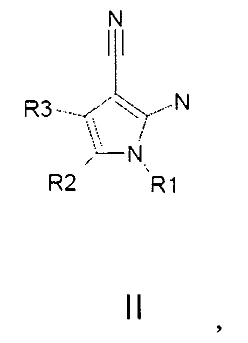 worin R1, R2 und R3 die in der Formel (I) angegebene Bedeutung haben,
mit Ameisensäure im 5-10-fachen Überschuss bei Refluxtemperatur 1 Stunde bis 2 Tage lang gerührt, dann das Gemisch in Eiswasser gegossen, das abgeschiedene Produkt abgetrennt, getrocknet, dann mit Phosporoxichlorid im 5-10-fachen Überschuss bei Refluxtemperatur 0,5 bis 4 Stunden lang versetzt, danach das Gemisch auf Eis gegossen und das abgeschiedene Imidoylchlorid der allgemeinen Formel (III) 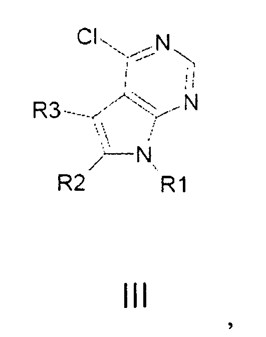 worin R1, R2 und R3 die in der Formel (I) angegebenen Bedeutungen haben, isoliert, getrocknet und eingedampft wird, darauf folgend
A) das Imidoylchlorid der allgemeinen Formel (III) in aprotischem Lösungsmittel gelöst und mit einer äquivalenten Menge Amin der allgemeinen Formel (II) oder (IV) 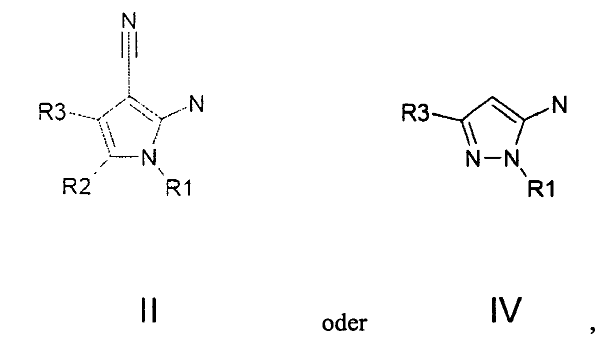 worin R1 und R3 die oben angegebene Bedeutung haben, versetzt, in einem 2-10-fachen molaräquivalenten Überschuss 0,5 bis 6 Stunden lang NaH zugesetzt wird, das hergestellte Gemisch auf Eis gegossen und das abgeschiedene Produkt isoliert und gereinigt wird, oder
B) das auf diese Weise hergestellte Imidoylchlorid der allgemeinen Formel (III) mit einem 2-10-fachen molaräquivalenten Überschuss in einem Medium aus polarem organischen Lösungsmittel mit Hydrazinhydrat versetzt wird, das Reaktionsprodukt in der organischen Phase aus dem Gemisch isoliert, die organische Phase getrocknet und eingedampft, dann mit einem apolaren Lösungsmittel verrieben wird, das auf diese Weise hergestellte Hydrazin-Derivat mit einem polaren organischen Lösungsmittel vermischt und mit einer äquivalenten Menge Aldehyd bei 20-120 °C 1 bis 12 Stunden lang versetzt wird, danach das Reaktionsprodukt isoliert wird, oder
C) das gemäß Variante B) hergestellte Hydrazin-Derivat mit einem polaren organischen Lösungsmittel vermischt und mit einer äquivalenten Menge Isatin versetzt, dann das Reaktionsprodukt isoliert wird.

## Revendications

1. Dérivés de 7H-pyrrolo[2,3-d]pyrimidine de formule générale (I), ainsi que leurs sels pharmaceutiquement acceptables, **caractérisé en ce que** dans la formule 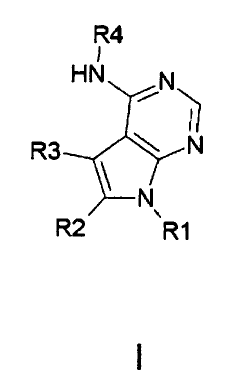
R1 est un groupement alkyle, aryle, hétéroaryle, aryl-alkyle de 1 à 4 atomes de carbone, hétéroaryl-alkyl de 1 à 4 atomes de carbone, morpholinoalkyle de 1 à 4 atomes de carbone ou dialkylaminoalkyle de 1 à 4 atomes de carbone,
R2, R3 sont indépendamment un atome d'hydrogène, un groupement méthyle, éthyle, propyle, isopropyle ou cyclopropyle, ou R2 et R3 forment ensemble un groupement tétraméthylène,
R4 est un groupement 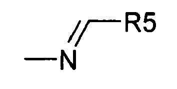 ou 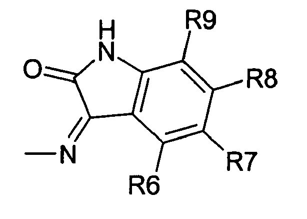 ou 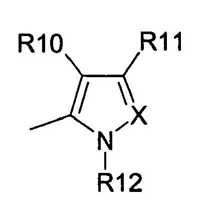 où
R5 est un cycle aromatique ou hétéroaromatique substitué ou non, où
R6, R7, R8 et R9 sont indépendamment un atome d'hydrogène ou d'halogène, ou un groupement nitro, amino, alkylamino, dialkylamino, hydroxy, méthoxy, éthoxy, isopropoxy ou sulfonyle,
R10 est un atome d'hydrogène ou un groupement nitrile,
R11 est un atome d'hydrogène ou un groupement méthyle, éthyle, propyle, isopropyle, tert.-butyl ou un cycle tétraméthylène lié au X,
R12 est alkyle, aryle, hétéroaryle, aryl-alkyle de 1 à 4 atomes de carbone, morpholinoalkyl de 1 à 4 atomes de carbone, dialkylaminoalkyl de 1 à 4 atomes de carbone,
X est un atome de carbone si R11 est un cycle tétraméthylène lié au X, pour le reste nitrogène, méthine, méthyl-méthine, éthyl-méthine, propyl-méthine, isopropyl-méthine, cyclopropyl-méthine, tert-butyl-méthine or phenyl- méthine

2. Les composés de la formule générale (I) selon la revendication 1, **caractérisé en ce que** leurs structures correspondent à la formule générale (Ia) 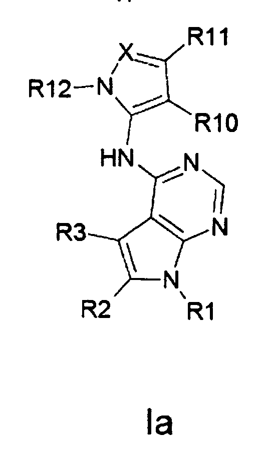 où R1, R2, R3, R10, R11, R12 et X sont les mêmes que dans la revendication 1.

3. Les composés de la formule générale (I) selon la revendication 1, **caractérisé en ce que** leurs structures correspondent à la formule générale (Ib) 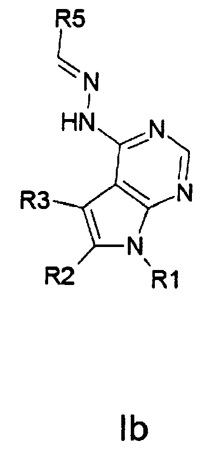 où R1, R2, R3 et R5 sont les mêmes que dans la revendication 1.

4. Les composés de la formule générale (I) selon la revendication 1, **caractérisé en ce que** leurs structures correspondent à la formule générale (Ic) 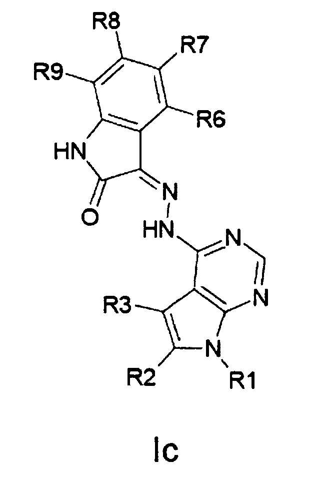 où R1, R2, R3, R6, R7, R8 et R9 sont les mêmes que dans la revendication 1.

5. Préparations pharmaceutiques, **caractérisé en c e** qu'elles contiennent des composés de formule générale (I) et des additifs thérapeutiquement acceptables.

6. Préparations pharmaceutiques selon la revendication 5, **caractérisé en ce qu'**elles peuvent être appliquées comme médicaments antiphlogistiques ou analgésiques.

7. Préparations pharmaceutiques selon la revendication 5, **caractérisé en ce qu'**elles peuvent être appliquées comme médicaments réduisant l'hyperalgésie neuropathique.

8. Préparations pharmaceutiques selon la revendication 5, **caractérisé en ce qu'**elles peuvent être appliquées comme médicaments réduisant la polyarthrite rhumatoïde.

9. Préparations pharmaceutiques selon la revendication 5, **caractérisé en ce qu'**elles peuvent être appliquées comme médicaments empêchant la destruction de l'os et du cartilage.

10. Préparations pharmaceutiques selon la revendication 5, **caractérisé en ce** q u ' elles peuvent être appliquées pour le traitement des maladies qui peuvent être liés aux procédés inflammatoires, e. g. asthme, eczéma ou psoriasis.

11. Procédé pour la préparation des dérivés de 7H-pyrrolo[2,3-d]pyrimidine de formule générale (I), **caractérisé en ce qu'**un composé de formule générale (II) 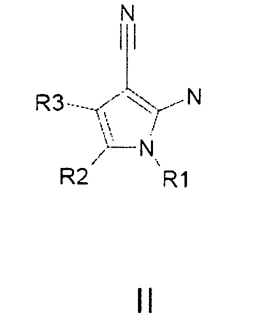 préparé de l'acétoïne avec de l'amine et du dinitrile de l'acide malonique en des quantités molaires équivalents, où R1, R2 et R3 sont les mêmes que dans la formule (I), le composé est mélangé avec de l'acide formique en un excès de poids de 5 à 10, à la température de reflux pendant un période de temps compris entre 1 heure et 2 jours, ensuite le mélange est versé dans de l'eau gelée, le produit précipité est séparé, le produit est séché puis mis en réaction avec de l'oxychlorure de phosphore en un excès de poids de 5 à 10, à la température de reflux pendant 0.5 à 4 heures, puis le mélange est versé sur de la glace, et le chlorure d'imidoyle précipité de formule générale (III) 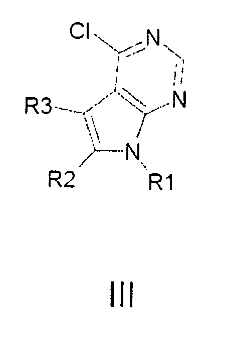 où R1, R2 et R3 sont les mêmes que dans la formule (I), est séparé, séché et évaporé, et ensuite
A) le chlorure d'imidoyle précipité de formule générale (III) est dissolu dans un solvant aprotique et mis en réaction avec une amine de formule générale (II) ou (IV) 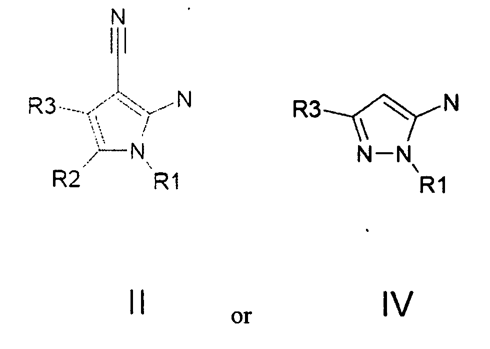 en une quantité équivalent, où R1 et R3 sont les mêmes qu'au dessus, ajoutant du NaH en excès molaire de 2 à 10 pendant 0.5 à 6 heurs, le mélange formé est versé sur de la glace et le produit précipité est séparé et purifié,
ou
B) le chlorure d'imidoyle de formule générale (III) préparé de cette manière est mis en réaction avec de l'hydrate d'hydrazine en excès molaire de 2 à 10 dans un milieu de solvant organique polaire, le produit de réaction dans la phase organique est séparé du mélange, la phase organique est séché et évaporé puis brisé avec un solvant apolaire, le dérivé de l'hydrazine préparé de cette manière est mélangé avec un solvant organique polaire et mis en réaction avec un aldéhyde en une quantité équivalent, à 20 to 120°C pendant 1 à 12 heurs, puis le produit de réaction est séparé,
ou
C) le dérivé de l'hydrazine préparé selon la version B) est mélangé avec un solvant organique polaire et mis en réaction avec de l'isatine en une quantité équivalent, puis le produit de réaction est séparé.
